# EUROPÄISCHE PATENTSCHRIFT

(11) **EP 0 750 602 B1**
(45) Veröffentlichungstag und Bekanntmachung des Hinweises auf die Patenterteilung: **19.08.1998**
(21) Anmeldenummer: 95912212.8
(22) Anmeldetag: 06.03.1995
(51) Int. Cl.: C07C 45/50, C07C 27/22, C07C 29/16, C07C 47/02

(54) **VERFAHREN ZUR HERSTELLUNG VON ALKOHOLEN UND/ODER ALDEHYDEN**
PROCESS FOR PRODUCING ALCOHOLS AND/OR ALDEHYDES
PROCEDE DE FABRICATION D'ALCOOLS ET/OU D'ALDEHYDES

(30) Priorität: 16.03.1994 DE 4408950
(43) Veröffentlichungstag der Anmeldung: 02.01.1997
(73) Patentinhaber: BASF AKTIENGESELLSCHAFT, 67056 Ludwigshafen (DE)
(72) Erfinder: KNEUPER, Heinz-Josef, D-68163 Mannheim (DE); ARON, Maik, D-67256 Weisenheim (DE); KORGITZSCH, Frank-Michael, D-67227 Frankenthal (DE); NILLES, Michael, D-67061 Ludwigshafen (DE); HARDER, Wolfgang, D-69469 Weinheim (DE); RÖPER, Michael, D-67157 Wachenheim (DE); PACIELLO, Rocco, D-67098 Bad Dürkheim (DE)
(86) Internationale Anmeldenummer: EP9500825
(87) Internationale Veröffentlichungsnummer: WO9525080

(56) Entgegenhaltungen:
- EP-A- 0 504 814
- WO-A-82/03856
- US-A- 3 984 478
- US-A- 4 329 521
- US-A- 4 400 547
- US-A- 4 740 626
- US-A- 5 041 685

## Beschreibung

Die vorliegende Erfindung betrifft ein Verfahren zur Herstellung von Alkoholen und/oder Aldehyden durch die Hydroformylierung von Olefinen mit mehr als 3' Kohlenstoffatomen, umfassend die Stufe der Hydroformylierung mittels eines homogen im Reaktionsmedium gelösten Rhodium-Katalysators, die Abtrennung des Rhodium-Katalysators vom Austrag der Hydroformylierungsreaktion und die Rückführung des vom Hydroformylierungsaustrag abgetrennten Rhodiums in die Hydroformylierungsstufe.

Die Hydroformylierung von Olefinen mit Kohlenmonoxid und Wasserstoff in Gegenwart von Übergangsmetall-Katalysatoren ist bekannt. Während α-Olefine sehr gut mit rhodiumhaltigen, phosphinmodifizierten Katalysatoren hydroformylierbar sind (vgl. J. Falbe, Ed: New Syntheses With Carbon Monoxide, Springer, Berlin 1980, S. 55 ff), ist dieses Katalysatorsystem für interne und interne, verzweigte Olefine sowie für Olefine mit mehr als 7 Kohlenstoffatomen wenig geeignet (vgl. Falbe, S. 95 ff). So werden interne Kohlenstoff-Kohlenstoff-Doppelbindungen nur sehr langsam in Gegenwart eines derartigen Katalysators hydroformyliert. Da die Abtrennung des Hydroformylierungsproduktes vom homogen im Reaktionssystem gelösten Katalysator in der Regel destillativ erfolgt und sich der Siedepunkt des bei der Hydroformylierung gebildeten Aldehyds mit zunehmender Kohlenstoffzahl und Kettenlänge auf Temperaturen erhöht, bei denen sich der rhodiumhaltige Katalysator zersetzt, ist diese Hydroformylierungsmethode für die Hydroformylierung von Olefinen mit mehr als 7 Kohlenstoffatomen nicht wirtschaftlich. Bei der Hydroformylierung polymerer Olefine, z.B. von Polyisobuten, kann der edelmetallhaltige Katalysator nicht in wiederverwendbarer Form wiedergewonnen werden.

Hingegen lassen sich interne und interne, verzweigte Olefine vorteilhaft mit sogenanntem "nacktem" Rhodium hydroformylieren, d.h. mit homogen im Hydroformylierungsmedium gelösten Rhodiumverbindungen, die nicht mit phosphorhaltigen Liganden, wie Phosphinen oder Phosphiten, modifiziert sind. Solche nicht mit Phosphinen oder Phosphiten modifizierte Rhodium-Katalysatoren und deren Eignung als Katalysator zur Hydroformylierung der zuvor genannten Klassen von Olefinen sind bekannt (siehe Falbe, S. 38 ff). Die Begriffe "nacktes Rhodium" oder "nackte Rhodium-Katalysatoren" werden in dieser Anmeldung für Rhodium-Hydroformylierungskatalysatoren gebraucht, die im Gegensatz zu herkömmlichen Rhodium-Hydroformylierungskatalysatoren unter den Bedingungen der Hydroformylierung nicht mit Liganden, insbesondere nicht mit phosphorhaltigen Liganden, wie Phosphin- oder Phosphit-Liganden, modifiziert sind. Als Liganden in diesem Sinne werden nicht Carbonyloder Hydrido-Liganden verstanden. Es wird in der Fachliteratur (s. Falbe, S. 38 ff) angenommen, daß die Rhodium-Verbindung HRh(CO)₄ die katalytisch aktive Rhodiumspezies bei der Hydroformylierung mit "nackten Rhodium-Katalysatoren" ist, obgleich dies aufgrund der vielen in der Hydroformylierungsreaktionszone nebeneinander ablaufenden Chemismen nicht eindeutig bewiesen ist. Lediglich der Einfachheit halber machen wir auch in dieser Anmeldung von dieser Annahme Gebrauch, ohne daß dadurch eine Einschränkung des Schutzumfangs der vorliegenden Anmeldung in Betracht zu ziehen wäre, falls sich zukünftig einmal eine andere Rhodiumspezies als die genannte, als die eigentlich katalytisch aktive, herausstellen sollte. Die "nackten Rhodium-Katalysatoren" bilden sich unter den Bedingungen der Hydroformylierungsreaktion aus Rhodium-Verbindungen, z.B. Rhodiumsalzen, wie Rhodium(III)chlorid, Rhodium(III)nitrat, Rhodium(III)acetat, Rhodium(II)acetat, Rhodium(III)sulfat oder Rhodium(III)ammoniumchlorid, aus Rhodiumchalkogeniden, wie Rhodium(III)oxid oder Rhodium(III)sulfid, aus Salzen von Rhodiumsauerstoffsäuren, beispielsweise den Rhodaten, aus Rhodium-Carbonyl-Verbindungen, wie Rh₄(CO)₁₂ und Rh₆(CO)₁₆ oder aus Organo-Rhodium-Verbindungen, wie Rhodiumdicarbonylacetonylacetonat, Cyclooctadien-Rhodiumacetat oder -chlorid in Gegenwart von CO/H₂-Gemischen, die gemeinhin als Synthesegas bezeichnet werden. Zur Durchführung von Hydroformylierungen mit "nacktem" Rhodium sei an dieser Stelle beispielhaft auf die folgenden Literaturstellen verwiesen: US-A 4 400 547; DE-A 33 38 340; DE-A 26 04 545; WO 82/03856; Chem. Ber. 102, 2238 (1969); Tetrahedron Lett. 29, 3261 (1968); Hydrocarbon Process. 85-86 (1975).

Allerdings hat auch die Hydroformylierung mit "nacktem" Rhodium den Nachteil, daß sich der thermolabile Rhodium-Katalysator (vgl. US-A 4 400 547) infolge der thermischen Belastung bei der destillativen Aufarbeitung des Hydroformylierungsproduktes teilweise zu metallischem Rhodium zersetzt, das sich an den Wandungen des Reaktors und der Rohrleitungen abscheidet. Das ausgefallene Rhodiummetall kann nicht wieder in die Hydroformylierungsreaktion zurückgeführt werden, da es unter den Hydroformylierungsbedingungen nicht in die katalytisch aktive Rhodiumverbindung umgewandelt werden kann. Die aus diesem chemischen Verhalten der "nackten Rhodium-Katalysatoren" resultierenden Rhodiumverluste haben bislang eine größere industrielle Anwendung dieses Verfahrens verhindert.

In DE-A 33 38 340 und US-A 4 400 547 werden Verfahren zur Hydroformylierung mittels "nackter Rhodium-Katalysatoren" beschrieben, bei denen zur Verhinderung der Rhodiumabscheidung dem Reaktionsaustrag der Hydroformylierung ein Phosphin oder Phosphit zugesetzt wird, welche den Rhodium-Katalysator durch die Bildung von Phosphin- und/oder Phosphit-Komplexen vor einer thermischen Zersetzung im Zuge der destillativen Aufarbeitung des Hydroformylierungsaustrags schützen. Nach Abschluß der Destillation wird der rhodiumhaltige Destillationssumpf mit einem Oxidationsmittel behandelt, wobei das Rhodium in katalytisch aktiver Form aus den betreffenden Phosphin- oder Phosphit-Komplexen freigesetzt wird und die Phosphin- und Phosphit-Liganden zu den entsprechenden, unter Hydroformylierungsbedingungen keine Rhodium-Komplexe bildenden Phosphinoxiden und Phosphaten oxidiert werden. Der oxidierte Destillationssumpf wird dann erneut als Katalysator zur Hydroformylierung eingesetzt. Die bei der Oxidation entstandenen oxidierten Phosphorverbindungen stören bei der Hydroformylierung in der Regel nicht, jedoch reichern sich verfahrensbedingt die oxidierten Phosphorverbindungen in diesem Hydroformylierungskreislauf an, weshalb ständig ein Teilstrom dieser Katalysatorlösung aus dem Hydroformylierungskreislauf ausgeschleust und durch frische Katalysatorlösung ergänzt werden muß. Die ausgeschleuste Katalysatorlösung muß einer gesonderten Prozedur zur Wiedergewinnung des darin enthaltenen Rhodiums unterzogen werden.

WO 82/03856 betrifft ein Verfahren zur Thermostabilisierung von unmodifizierten, also "nackten Rhodium-Katalysatoren", in dem der Austrag aus der Hydroformylierungsreaktion mit einem sauerstoffhaltigen Gas behandelt wird, wodurch die gebildeten Aldehyde z.T. zu den entsprechenden Carbonsäuren oxidiert werden, welche mit dem Rhodium-Katalysator bei der destillativen Aufarbeitung thermostabile Rhodium-Carboxylate bilden, die wieder als Katalysatoren zur Hydroformylierung verwendet werden können. Nachteilig an diesem Verfahren ist die Verringerung der Ausbeute, als Folge der partiellen Oxidation der Produktaldehyde zu Carbonsäuren. Darüber hinaus ist dieses Verfahren auf solche Hydroformylierungen beschränkt, bei denen destillierbare Produkte gebildet werden: So kann nach diesem Verfahren z.B. der Rhodium-Katalysator nicht vom Hydroformylierungsprodukt des Polyisobutens abgetrennt werden.

Zur Vermeidung von Rhodiumverlusten wurde gemäß US-A 3 984 478 ein Hydroformylierungsverfahren entwickelt, bei dem die Hydroformylierung in Gegenwart eines gegebenenfalls sulfonierten Phthalocyanins durchgeführt wird. Da die hierbei gebildeten Rhodium-Phthalocyanin-Komplexe z.T. schwer löslich oder nur in Wasser, nicht jedoch im organischen Hydroformylierungsmedium löslich sind, wird die Hydroformylierung wahlweise in Gegenwart der festen Rhodium-Phthalocyanine oder im Zwei-Phasen-System mit Wasser durchgeführt. Allerdings ist die koordinative Bindung des Rhodiums zum Phthalocyanin in diesen Komplexen sehr fest, so daß das Rhodium auch unter den Hydroformylierungsbedingungen ans Phthalocyanin gebunden bleibt. Als Folge hiervon findet die Hydroformylierungsreaktion nur an der Grenzfläche Hydroformylierungsmedium/festes Phthalocyanin bzw. an der Grenzfläche zur wäßrigen Rhodium-Phthalocyanin-Komplexlösung statt, wodurch die Reaktionsgeschwindigkeit und damit auch die Raum-Zeit-Ausbeute der Hydroformylierungsreaktion so niedrig wird, daß sich dieses Verfahren nicht wirtschaftlich betreiben läßt.

Zur direkten Herstellung interner, d.h. verzweigtkettiger Aldehyde, sogenannten Isoaldehyden, durch die Hydroformylierung von α-Aldehyden, also Aldehyden mit endständiger Doppelbindung, gibt es bislang kein wirtschaftlich zufriedenstellendes Verfahren. Bott (Fette, Seifen, Anstrichmittel, 76, 443 (1974)) beschreibt die Synthese von internen Aldehyden aus α-Olefinen durch deren Isomerisierung mittels Kobaltoctacarbonyl (Co₂(CO)₈) unter einer Kohlenmonoxid-Atmosphäre bei 190°C oder an einem Natrium auf Aluminiumoxid-Katalysator und die nachfolgende Hydroformylierung der internen Olefine mittels Rhodium-Triphenylphosphin-Homogenkatalysatoren. Nachteilig ist hierbei die Verwendung zweier verschiedener Katalysatoren für die einzelnen Reaktionsschritte. Zudem reagieren interne Olefine mit den Rhodium-Triphenylphosphin-Katalysator für technische Zwecke zu langsam.

Nach Fell et al (Tetrahedron Lett., 29, 3261 (1968)) wird Octen-1 zu internen Olefinen an einem aus Rh₂O₃ unter 200 bar (CO/H₂ (1/1)) Kaltdruck und bei 150°C in Hexan hergestellten Katalysator, welcher durch mehrmaliges Aufpressen von Kohlenmonoxid von Wasserstoff befreit worden ist, bei 100 bar Kohlenmonoxid-Druck und 190°C isomerisiert. Nachteilig an diesem Verfahren ist die aufwendige Katalysatorherstellung, die Verwendung eines inerten Lösungsmittels, welches einen erheblichen Teil des zur Verfügung stehenden Hochdruck-Reaktionsraumes in Anspruch nimmt und daher die Raum-Zeit-Ausbeute erniedrigt, sowie der Einsatz unterschiedlicher Reaktionsgase in der Isomerisierungsstufe (Kohlenmonoxid) und in der nachfolgenden Hydroformylierungsstufe (Synthesegas). Weiterhin wurde von Fell et al das Problem der Katalysatorstabilität und -regenerierung nicht gelöst. Dieses Verfahren ist somit ebenfalls unwirtschaftlich.

Eine Studie über kinetische und mechanistische Untersuchungen zur Isomerisierung von α-Olefinen zu internen Olefinen und die Hydroformylierung ist in Kogyo Kagaku Zasski, 72, 671 (1969) veröffentlicht.

Der vorliegenden Erfindung lag daher die Aufgabe zugrunde, ein Verfahren zur Herstellung von Aldehyden aus langkettigen und/oder verzweigten Olefinen mit Hilfe "nackter Rhodium-Katalysatoren" zu finden, mit dem zum einen die Probleme der Abscheidung metallischen Rhodiums bei der destillativen Aufarbeitung des Hydroformylierungsproduktes sowie der Rhodium-Katalysator-Abtrennung von nicht destillierbaren Produktaldehyden zufriedenstellend gelöst werden können. Es sollte zu diesem Zweck nach einem Hydroformylierungsverfahren gesucht werden, in dem sich Komplex-Liganden reversibel und in Abhängigkeit vom Druck der Kohlenmonoxid-Wasserstoff-Gasmischung an den Rhodium-Katalysator koordinativ binden, so daß dieser bei einer extraktiven Aufarbeitung stabilisiert und extrahierbar wird. Nach Rückführung in die Hydroformylierungsreaktion sollten die so gebildeten Komplexe unter dem angewandten Reaktionsdruck in Gegenwart der Kohlenmonoxid-Wasserstoff-Gasmischung reversibel dekomplexieren und die freigesetzte Rhodiumverbindung wieder die katalytischen Eigenschaften des "nackten Rhodiums" annehmen. Ziel war es dabei insbesondere eine Schädigung des Komplex-Liganden im Zuge der Hydroformylierungsreaktion zu vermeiden, die Verluste an Rhodium im Zuge der Extraktion zu minimieren und das Verfahren so auszugestalten, daß sich die Hydroformylierung interner Olefine wirtschaftlich und in hohen Raum-Zeit-Ausbeuten durchführen läßt.

Eine weitere Aufgabe war es, ein Verfahren zu finden, das die Herstellung von Isoaldehyden aus α-Olefinen auf wirtschaftliche Weise gestattet. Dabei sollten insbesondere die zuvor geschilderten Nachteile des Standes der Technik vermieden werden.

Dementsprechend wurde ein Verfahren zur Herstellung von Alkoholen und/oder Aldehyden durch die Hydroformylierung von Olefinen mit mehr als 3 Kohlenstoffatomen, umfassend die Stufe der Hydroformylierung mittels eines homogen im Reaktionsmedium gelösten Rhodium-Katalysators, die Abtrennung des Rhodium-Katalysators vom Austrag der Hydroformylierungsreaktion und die Rückführung des vom Hydroformylierungsaustrag abgetrennten Rhodiums in die Hydroformylierungsstufe, gefunden, das dadurch gekennzeichnet ist, daß man den Rhodium-Katalysator aus dem Hydroformylierungsaustrag mittels einer wäßrigen Lösung eines stickstoffhaltigen Komplexbildners aus der Gruppe der gegebenenfalls substituierten, sulfonierten oder sulfonierte Substituenten tragenden Pyridine, Chinoline, 2,2'-Bipyridine, 1,10-Phenanthroline, 2,2'-Bichinoline, 2,2',6',2''-Terpyridine und Porphine, und/oder aus der Gruppe der carboxylierten Pyridine, der carboxylierten Chinoline, der gegebenenfalls substituierten, carboxylierten oder carboxylierte Substituenten tragenden 2,2'-Bipyridine, 1,10-Phenanthroline, 2,2'-Bichinoline, 2,2',6',2"-Terpyridine und Porphine in die wäßrige Phase extrahiert und aus dem extrahierten Hydroformylierungsaustrag den Alkohol und/oder Aldehyd isoliert, den wäßrigen rhodiumhaltigen Extrakt einer Vorcarbonylierungsstufe zuführt und ihn in der Vorcarbonylierungsstufe in Gegenwart einer im wesentlichen wasserunlöslichen, organischen Flüssigkeit und in Gegenwart von Kohlenmonoxid, Synthesegas oder eines Kohlenmonoxid-haltigen Gasgemisches bei einem Druck von 50 bis 1000 bar und einer Temperatur von 50 bis 180°C einer Vorcarbonylierung unterzieht, den Austrag aus der Vorcarbonylierungsstufe in eine den Hauptteil des Rhodiums enthaltende organische Phase und in eine den Komplexbildner enthaltende wäßrige Phase trennt und die organische Phase zur Hydroformylierung des Olefins bei 50 bis 1000 bar und einer Temperatur von 50 bis 180°C der Hydroformylierungsstufe zuführt.

Erfindungsgemäß werden die bei der Hydroformylierung mit "nacktem Rhodium" erhaltenen rhodiumhaltigen Hydroformylierungsausträge mit wasserlöslichen, stickstoffhaltigen Komplexbildnern, vorzugsweise mehrzähnigen Komplexbildnern, versetzt, die mit dem Rhodium-Katalysator Komplexe bilden, welche hydrophil sind und infolge ihrer guten Wasserlöslichkeit mit Wasser aus dem organischem Medium des Hydroformylierungsaustrags extrahiert werden können. Nach der extraktiven Abtrennung des im Hydroformylierungsaustrag enthaltenen Rhodium-Katalysators in Form eines wasserlöslichen Komplexes mit dem erfindungsgemäß eingesetzten Komplexbildner, kann das Hydroformylierungsprodukt auf an sich übliche Weise aufgearbeitet werden, beispielsweise indem man das Hydroformylierungsprodukt aus dem organischen Extrakt destillativ isoliert oder indem man leichter flüchtige organische Bestandteile des Hydroformylierungsaustrags vom schwerer flüchtigen oder gegebenenfalls sogar undestillierbaren Hydroformylierungsprodukt abdestilliert. Der wäßrige Extrakt des Hydroformylierungsaustrags, welcher den nunmehr vom stickstoffhaltigen Komplexbildner komplexierten Rhodium-Katalysator enthält, wird einer Verarbeitungsstufe zugeleitet, in der der komplexierte Rhodium-Katalysator in Gegenwart einer im wesentlichen wasserunlöslichen, organischen Flüssigkeit und in Gegenwart von Kohlenmonoxid, Synthesegas oder eines Kohlenmonoxid-haltigen Gasgemisches bei einem Druck von im allgemeinen 50 bis 1000 bar, vorzugsweise von 70 bis 500 bar und besonders bevorzugt von 100 bis 400 bar und bei einer Temperatur von 50 bis 180°C, vorzugsweise von 70 bis 160°C, insbesondere von 90 bis 140°C, carbonyliert wird. Durch die Carbonylierung wird das Rhodium aus dem hydrophilen Komplex mit dem stickstoffhaltigen Komplexbildner gelöst, und die sich dabei bildende, lipophile Rhodiumcarbonylverbindung migriert als "nacktes" Rhodium in die wasserunlösliche, organische Flüssigkeit. Dieser Schritt wird als "Vorcarbonylierung" bezeichnet, da die Carbonylierung des Rhodiums nicht im Hydroformylierungsreaktor selbst, sondern eben in der diesem vorgeschalteten Vorcarbonylierungsstufe erfolgt.

Der Austrag aus der Vorcarbonylierungsstufe kann leicht, z.B. in einem Phasenabscheider in eine den Hauptteil des Rhodiums in Form einer Rhodiumcarbonylverbindung enthaltende organische Phase und in eine den Hauptteil des Komplexbildners enthaltende wäßrige Phase getrennt werden. Die organische Phase wird anschließend der Hydroformylierungsstufe zugeführt, in der das in dieser Katalysatorlösung enthaltene "nackte" Rhodium die Hydroformylierung des zu hydroformylierenden Olefins katalysiert. Die den Hauptteil des Komplexbildners enthaltende, wäßrige Phase kann anderweitig verwendet werden, beispielsweise vorteilhaft zur Extraktion des Rhodium-Katalysators aus dem Hydroformylierungsaustrag.

Die Verwendung einer solchen Vorcarbonylierungsstufe führt zu erheblichen Vorteilen. Da die Carbonylierung im allgemeinen schneller und unter milderen Bedingungen als die Hydroformylierungsreaktion erfolgt, kann zum einen der stickstoffhaltige Komplexbildner weniger stringenten Reaktionsbedingungen während einer kürzeren Verweilzeit ausgesetzt und auf diese Weise geschont werden, zum anderen kann bei gleicher Raum-Zeit-Ausbeute mit kleineren Reaktordimensionen gearbeitet werden, da die wäßrige Phase nicht mehr in den Hydroformylierungsreaktor gelangt.

Die Vorcarbonylierung kann mit Hilfe von Kohlenmonoxid, Synthesegas oder eines Kohlenmonoxid-haltigen Gasgemisches durchgeführt werden. Unter "Synthesegas" werden CO/H₂-Gasgemische verstanden, in den Kohlenmonoxid und Wasserstoff im allgemeinen in einem Molverhältnis von 1:5 bis 5:1, vorzugsweise von 4:6 bis 6:4 vorliegen. Als Kohlenmonoxid-haltige Gasgemische werden im Sinne dieser Anmeldung andere Kohlenmonoxid-haltige Gasgemische verstanden, die nicht unter den Begriff "Synthesegas" fallen, beispielsweise CO/H₂-Gemische mit von Synthesegas verschiedener Zusammensetzung oder Gemische des Kohlenmonoxids mit anderen, unter den Reaktionsbedingungen inerten Gasen, wie Stickstoff, Edelgasen oder niederen Kohlenwasserstoffen, wie Methan, Ethan, Propan oder Butan.

Als im wesentlichen wasserunlösliche organische Flüssigkeit können erfindungsgemäß eine Vielzahl unter den Reaktionsbedingungen der Vorcarbonylierungsstufe und der Hydroformylierungsstufe inerten Flüssigkeiten verwendet werden, wobei "inert" bedeutet, daß diese Flüssigkeiten nicht den Ablauf der Vorcarbonylierung oder der Hydroformylierung beeinträchtigen.

Als derartige organische Flüssigkeiten können beispielsweise Kohlenwasserstoffe verwendet werden. Vorzugsweise werden aber Aldehyde oder Alkohole oder Gemische aus Aldehyden und Alkoholen verwendet. Beispielsweise kann zu diesem Zweck ein Teil des rohen Austrags aus der Hydroformylierungsstufe verwendet werden, es können aber auch die in der Hydroformylierungsstufe gebildeten und nachfolgend isolierten Aldehyde oder Alkohole oder deren Gemische verwendet werden. Es besteht bezüglich der Art der als wasserunlösliche, organische Flüssigkeit in der Vorcarbonylierungsstufe verwendeten Aldehyde praktisch keine Beschränkung. Vorzugsweise werden aber solche Aldehyde oder Alkohole verwendet, wie sie bei der Hydroformylierung des zu hydroformylierenden Olefins entstehen.

Als im wesentlichen wasserunlösliche, organische Flüssigkeit können in der Vorcarbonylierungsstufe auch sogenannte Hochsieder eingesetzt werden. Diese sind hochsiedende Kondensationsprodukte von Aldehyden, die im Zuge der Hydroformylierung als Nebenprodukte entstehen. Es handelt sich hierbei naturgemäß im allgemeinen um Vielkomponentenmischungen. In US-A 4 148 830 wird die chemische Natur derartiger Hochsiedergemische beispielhaft erläutert. Derartige Hochsiedergemische sind auch im Handel erhältlich, beispielsweise unter der Bezeichnung Texanol® von der Firma Eastman.

Besonders bevorzugt werden im erfindungsgemäßen Verfahren Olefine als im wesentlichen wasserunlösliche, organische Flüssigkeit in die Vorcarbonylierungsstufe eingesetzt. Obgleich bezüglich der Art des in der Vorcarbonylierung eingesetzten Olefins prinzipiell keine Beschränkung besteht, werden vorzugsweise solche Olefine eingesetzt, wie sie in der nachfolgenden Hydroformylierungsstufe eingesetzt werden.

Es kann sich auch als vorteilhaft erweisen, den gesamten Olefinzulauf für die Hydroformylierungsstufe zunächst durch die Vorcarbonylierungsstufe zu schicken. Wird in der Vorcarbonylierungsstufe Synthesegas als Carbonylierungsagens verwendet, kann das zugeführte Olefin je nach den gewählten Bedingungen in der Vorcarbonylierung bereits in geringen Mengen hydroformyliert werden. Werden Kohlenmonoxid oder Kohlenmonoxid-haltige Gasgemische als Carbonylierungsagens benutzt, so können sich Acyl-Komplexe des carbonylierten Rhodiums mit dem Olefin bilden, wodurch eine zusätzliche Stabilisierung des homogen gelösten Rhodiums erzielt werden kann.

Es wurde überraschenderweise festgestellt, daß beim Einsatz von α-Olefinen in die Vorcarbonylierungsstufe, eine Isomerisierung der α-Olefine zu internen Olefinen stattfinden kann. Somit können α-Olefine erfindungsgemäß zu internen Olefinen isomerisiert und diese in der nachfolgenden Hydroformylierungsstufe zu internen, also verzweigten, Aldehyden hydroformyliert werden. Dies ist besonders vorteilhaft, da α-Olefine verglichen mit internen Olefinen auf dem Markt in größeren Mengen zur Verfügung stehen als interne Olefine, α-Olefine zudem preiswerter erhältlich sind als interne Olefine und verzweigte Aldehyde und verzweigte Alkohole gesuchte Zwischenprodukte zur Herstellung von verzweigten Carbonsäuren, Alkoholen und Aminen sind, welche wiederum in großem Umfang z.B. als Zusätze für Wasch- und Reinigungsmittel und zur Herstellung von biologisch abbaubaren Tensiden verwendet werden.

Zur Herstellung von verzweigten Alkoholen und/oder Aldehyden aus α-Olefinen gemäß dem erfindungsgemäßen Verfahren wird zweckmäßigerweise der α-Olefinzulauf durch die Vorcarbonylierungsstufe geschickt. Die Vorcarbonylierung und simultan dazu die Isomerisierung des α-Olefins zum internen Olefin wird im allgemeinen bei Temperaturen von 100 bis 180°C, vorzugsweise bei 120 bis 160°C, besonders bevorzugt bei 130 bis 150°C und bei einem Druck von 50 bis 1000 bar, vorzugsweise 70 bis 500 bar und besonders bevorzugt bei 100 bis 400 bar durchgeführt. Die zur vollständigen Isomerisierung des α-Olefins erforderliche Verweilzeit des α-Olefins in der Vorcarbonylierungsstufe ist im allgemeinen von den darin angewandten Reaktionsbedingungen abhängig und wird zweckmäßigerweise durch einen Vorversuch bestimmt.

Selbstverständlich können nach dem erfindungsgemäßen Verfahren auch α-Olefine zu n-Aldehyden hydroformyliert werden, beispielsweise indem man das α-Olefin unter Umgehung der Vorcarbonylierungsstufe in den Hydroformylierungsreaktor einleitet oder indem man die Reaktionsbedingungen in der Vorcarbonylierungsstufe so wählt, daß das der Vorcarbonylierungsstufe zugeführte α-Olefin nicht in nennenswertem Umfang isomerisiert wird.

Die Vorcarbonylierungsstufe kann aus einem oder mehreren, parallel oder hintereinander geschalteten Reaktoren bestehen. Bei einer diskontinuierlichen Betriebsweise können hierzu herkömmliche Rührautoklaven und bei einer kontinuierlichen Betriebsweise Kaskaden-Rührautoklaven oder Rohrreaktoren, die zur Durchmischung des Reaktionsgutes geeigneten Vorrichtungen enthalten, verwendet werden.

Der Austrag aus der Vorcarbonylierungsstufe wird in einer geeigneten Vorrichtung, z.B. einem Phasenabscheider, in eine wäßrige und eine organische Phase getrennt. Die Phasenabscheidung kann unter Druck, beispielsweise unter dem Betriebsdruck der Vorcarbonylierungsstufe oder bei Atmosphärendruck, nach vorausgegangener Entspannung des Austrags aus der Vorcarbonylierung erfolgen. Da sowohl die Vorcarbonylierung als auch die Hydroformylierung unter erhöhtem Druck erfolgen, wird die Phasentrennung vorteilhaft ebenfalls unter Druck vorgenommen.

Die so aus dem Austrag der Vorcarbonylierungsstufe abgetrennte organische Phase, die das zur Katalyse der Hydroformylierung benötigte "nackte" Rhodium und je nach Art der in der Vorcarbonylierungsstufe verwendeten wasserunlöslichen, organischen Flüssigkeit das zu hydroformylierende Olefin oder eine andere geeignete organische Flüssigkeit enthält, kann der Hydroformylierungsstufe zugeführt werden. Wurde keine Entspannung und Entgasung des Vorcarbonylierungsaustrags vorgenommen, enthält die organische Phase weiterhin das in der Vorcarbonylierungsstufe verwendete gasförmige Carbonylierungsagens im wesentlichen in gelöster Form.

Die Hydroformylierung mit Hilfe des in der Vorcarbonylierungsstufe erzeugten "nackten" Rhodium-Katalysator wird in Gegenwart von Synthesegas durchgeführt. Erforderlichenfalls wird der Hydroformylierungsstufe noch das zu hydroformylierende Olefin zugeführt, falls dieses der Hydroformylierungsstufe nicht schon mit der organischen Phase aus dem Vorcarbonylierungsaustrag zugeführt wurde.

Die Hydroformylierung wird im allgemeinen bei Temperaturen von 60 bis 180°C, vorzugsweise 80 bis 140°C und besonders bevorzugt bei 90 bis 130°C und bei einem Druck von im allgemeinen 50 bis 1000 bar, vorzugsweise bei 70 bis 500 bar, insbesondere bei 100 bis 400 bar durchgeführt. Die Hydroformylierung erfolgt ansonsten unter Bedingungen, wie sie üblicherweise bei Hydroformylierungen mit "nacktem" Rhodium angewandt werden und wie sie beispielsweise in der eingangs zitierten Literatur, betreffend die Hydroformylierung mit "nacktem" Rhodium, beschrieben sind.

In Abhängigkeit von den in der Hydroformylierungsstufe angewandten Druck- und Temperaturbedingungen und der Synthesegaszusammensetzung kann das Produktverhältnis Alkohol/Aldehyd im Hydroformylierungsaustrag beeinflußt werden. Beispielsweise wird bei der Hydroformylierung von Trimerpropylen bei jeweils gleichen Synthesegaszusammensetzungen - CO/H₂-Molverhältnis 50/50, 40/60 bzw. 60/40 - bei 130°C und einem Druck von 280 bar ein Aldehyd/Alkohol-Molverhältnis von jeweils 93/7 erhalten. Bei Erhöhung der Temperatur von 130°C auf 150°C verändert sich das Aldehyd/Alkohol-Molverhältnis im Hydroformylierungsaustrag in Abhängigkeit von der Synthesegaszusammensetzung - CO/H₂-Molverhältnis 50/50, 40/60 bzw. 60/40 - auf 76/24, 67/33 bzw. 82/18.

Die Hydroformylierung kann in An- oder Abwesenheit von organischen Lösungsmitteln durchgeführt werden. Besonders vorteilhaft ist die Verwendung organischer Lösungsmittel, insbesondere bei der Hydroformylierung langkettiger oder polymerer Olefine. Als Lösungsmittel können die im Hydroformylierungsverfahren üblicherweise eingesetzten Lösungsmittel benutzt werden, beispielsweise hochsiedende aromatische und aliphatische Kohlenwasserstoffe oder auch hochsiedende Aldehyd-Kondensationsprodukte, die im Zuge der Hydroformylierungsreaktion als Nebenprodukt als Folge der Kondensation der Produktaldehyde entstehen.

Der Austrag aus der Hydroformylierungsstufe wird vor seiner Extraktion mit der wäßrigen Lösung des stickstoffhaltigen Komplexbildners zweckmäßigerweise entspannt. Die Extraktion des Hydroformylierungsaustrags wird im allgemeinen bei Temperaturen von 80 bis 140°, vorzugsweise von 90 bis 130°C, insbesondere 100 bis 120°C und bei einem Druck von im allgemeinen 1 bis 20 bar, vorzugsweise 1 bis 10 bar und besonders bevorzugt von 1 bis 5 bar durchgeführt. Die Extraktion kann an der Luft oder unter einer Inertgasatmosphäre durchgeführt werden, beispielsweise einer Stickstoff-, Wasserstoff- oder Argonatmosphäre, es kann aber auch vorteilhaft sein, dem verwendeten Inertgas zusätzlich Kohlenmonoxid oder Synthesegas beizumischen oder die Extraktion in Gegenwart von Kohlenmonoxid durchzuführen.

Zur Extraktion des Rhodium-Katalysators aus dem Hydroformylierungsaustrag kann eine frische wäßrige Lösung des Komplexbildners verwendet werden, vorzugsweise wird hierfür aber die bei der Phasentrennung des Vorcarbonylierungsaustrag erhaltene, den gelösten Komplexbildner enthaltende wäßrige Phase verwendet, die zu diesem Zweck in die Extraktionsstufe zurückgeführt wird.

Bei der Extraktion wird im allgemeinen ein Volumenverhältnis wäßrige/organische Phase von im allgemeinen 0,2 bis 2, vorzugsweise von 0,3 bis 1, eingestellt. Das Molverhältnis stickstoffhaltiger Komplexbildner/Rhodium beträgt bei der Extraktion im allgemeinen 5:1 bis 10000:1, vorzugsweise 10:1 bis 5000:1, insbesondere 50:1 bis 1000:1.

Zur Extraktion des Hydroformylierungsaustrags mit der wäßrigen Lösung des Komplexbildners sind praktisch alle Flüssig-flüssig-Extraktionsapparaturen geeignet, beispielsweise Mixer-Settler-Apparaturen, Blasensäulen oder Gegen- oder Gleichstromextraktionskolonnen, wobei diese noch mit zusätzlichen Einbauten zur besseren Durchmischung von wäßriger und organischer Phase versehen sein können, beispielsweise mit Siebböden, Füllkörpern oder statischen Mischern. Die Extraktion des Rhodium-Katalysators aus dem Hydroformylierungsaustrag kann einstufig durchgeführt werden, vorzugsweise wird eine Mehrstufenextraktion angewandt, beispielsweise eine Zwei- oder Dreistufenextraktion, wobei die wäßrige, den Komplexbildner enthaltende Phase im Gleichstrom oder, besonders bevorzugt, im Gegenstrom bezüglich der organischen Phase geführt werden kann.

Nach beendeter Extraktion kann der vom Rhodium-Katalysator befreite Hydroformylierungsaustrag auf an sich herkömmliche Weise, beispielsweise destillativ zur Isolierung der darin enthaltenen Wertprodukte - Alkohole und oder Aldehyde - aufgearbeitet werden.

Eine vorteilhafte Ausgestaltung des erfindungsgemäßen Verfahrens wird in Fig. schematisch dargestellt und wird im folgenden erläutert. An sich selbstverständliche Anlagendetails, die zu der Veranschaulichung des erfindungsgemäßen Verfahren nicht erforderlich sind, wurden aus Gründen der Übersichtlichkeit nicht in Fig. aufgenommen. Die in Fig. dargestellte Ausgestaltung des erfindungsgemäßen Verfahrens umfaßt die Verfahrensstufen der Hydroformylierung, eine zweistufige Gegenstromextraktion des Hydroformylierungsaustrags mittels Mixer-Settler-Apparaturen und die Vorcarbonylierungsstufe. Es versteht sich von selbst, daß an Stelle der Mixer-Settler-Apparaturen auch andere der oben erwähnten Extraktionsapparaturen eingesetzt werden können.

In der Ausgestaltung des erfindungsgemäßen Verfahrens gemäß Fig. wird der Hydroformylierungsaustrag aus dem Hydroformylierungsreaktor 1 über Leitung 2 nach Entspannung und gegebenenfalls nach Zufuhr von Inertgas (nicht eingezeichnet) in die Extraktionsstufe A, umfassend die Mixer-Settler-Apparatur 3/4, eingetragen und dort mit der über Leitung 19 zugeführten wäßrigen Lösung des stickstoffhaltigen Komplexbildners aus Extraktionsstufe C (Mixer-Settler-Apparatur 6/7) extrahiert. Beim Anfahren der Anlage oder zum Zwecke der Ergänzung der Komplexbildnerlösung kann frische Komplexbildnerlösung über einen nicht in Fig. eingezeichneten Einlaß z.B. dem Mixer 3 zugeführt werden. Das im Mixer 3 enthaltene Extraktionsgemisch wird im Settler 4 in eine erste organische und eine erste wäßrige Phase aufgetrennt. Die erste wäßrige Phase wird über Leitung 8 in den Vorcarbonylierungsreaktor geleitet, wohingegen die erste organische Phase über Leitung 5 der Extraktionsstufe C zugeführt wird. Vor Einleitung in den Vorcarbonylierungsreaktor 11 wird die erste wäßrige phase noch in geeigneten Mischapparaturen über die Zuläufe 9 und 10 mit einer im wesentlichen wasserunlöslichen, organischen Flüssigkeit, beispielsweise rohem Hydroformylierungsaustrag, Texanol® oder vorzugsweise dem zu hydroformylierenden Olefin, und dem Carbonylierungsagens, also Kohlenmonoxid, Synthesegas oder einem geeigneten Kohlenmonoxid-haltigen Gasgemisch, vorzugsweise mit Kohlenmonoxid oder Synthesegas, vermischt. Es ist grundsätzlich auch möglich die über die Leitungen 9 und 10 zugeführten Einsatzstoffe direkt in den Vorcarbonylierungsreaktor 11 einzuleiten. Im Vorcarbonylierungsreaktor 10 wird das an den stickstoffhaltigen Komplexbildner gebundene, in der wäßrigen Phase befindliche Rhodium unter den angegebenen Bedingungen carbonyliert und der sich dabei bildende, lipophile "nackte" Rhodium-Katalysator migriert in die organische Phase. Der Austrag aus dem Vorcarbonylierungsreaktor 11 wird über Leitung 12, vorzugsweise ohne vorherige Entspannung, in den Phasenabscheider 13 geleitet und dort in eine zweite organische und eine zweite wäßrige Phase getrennt (Phasenabscheidung B).

Die zweite organische Phase, die außer der wasserunlöslichen, organischen Flüssigkeit noch das zur Katalyse der Hydroformylierung benötigte "nackte" Rhodium und gegebenenfalls überschüssiges Carbonylierungsagens gelöst enthält, wird über Leitung 14 in den Hydroformylierungsreaktor 1 geleitet. Über Leitung 15 wird dem Hydroformylierungsreaktor Synthesegas zugeleitet, welches alternativ auch direkt in Hydroformylierungsreaktor 1 geleitet werden kann. Wurde im Vorcarbonylierungsreaktor als wasserunlösliche, organische Flüssigkeit kein Olefin verwendet, so kann das zu hydroformylierende Olefin entweder direkt über Leitung 16 in den Hydroformylierungsreaktor 1 eingeleitet werden oder vorher über einen nicht in Fig. eingezeichneten Einlaß dem Strom in Leitung 14 zugemischt werden. Im Hydroformylierungsreaktor wird das Olefin unter den angegebenen Bedingungen zu den entsprechenden Alkoholen und/oder Aldehyden hydroformyliert.

Die zweite wäßrige Phase aus Phasenabscheidung B, die die an Rhodium abgereicherte Lösung des stickstoffhaltigen Komplexbildners enthält, wird über Leitung 17 nach vorheriger Entspannung dem Mixer 6 zugeführt. In Extraktionsstufe C, umfassend den Mixer 6 und den Settler 7, wird die erste organische Phase aus Extraktionsstufe A mit der zweiten wäßrigen Phase aus Phasenabscheidung B nochmals extrahiert, um Restmengen von Rhodium aus der ersten organischen Phase zu entfernen. Die im Mixer 6 enthaltene Extraktionsmischung wird im Settler 7 in eine dritte organische und eine dritte wäßrige Phase getrennt. Die nunmehr von Rhodium befreite dritte organische Phase wird über Leitung 18 zur weiteren Aufarbeitung zwecks Isolierung der Wertprodukte - Alkohol und/ oder Aldehyd - ausgetragen. Die dritte wäßrige Phase aus Extraktion C wird über Leitung 19 in die Extraktionsstufe A geleitet, womit der Kreislauf geschlossen ist.

Die Erstbefüllung des Reaktors mit Rhodium kann durch Einführung einer Lösung oder Suspension des Rhodiumkatalysators oder eine der zur Herstellung des Rhodium-Katalysators geeigneten, eingangs erwähnten Vorläuferverbindungen, beispielsweise in den Vorcarbonylierungsreaktor 11 oder in den Hydroformylierungsreaktor 1, erfolgen. Gleiches gilt für gegebenenfalls erforderliche Ergänzungen von verbrauchtem Katalysator. Es ist auch möglich das Rhodium über Leitung 20 oder anderen, in Fig. nicht eingezeichneten Einlässen, beispielsweise über einen Einlaß an Leitung 8, in die Anlage einzuleiten.

Als Komplexbildner, die mit dem im Austrag der Hydroformylierungsreaktion gelösten Rhodium-Katalysator wasserlösliche Komplexe bilden, werden vorzugsweise sulfonierte, stickstoffhaltige Komplexbildner, wie sulfonierte Pyridine oder sulfonierte Chinoline und/oder wasserlösliche carboxylierte Pyridine oder Chinoline, verwendet. Vorzugsweise werden mehrzähnige, insbesondere zwei-, drei- oder vierzähnige, sulfonierte stickstoffhaltige Komplexbildner verwendet, besonders bevorzugt sind 2,2'-Bipyridin-Sulfonate, 2,2'-Bichinolin-Sulfonate, 1,10-Phenanthrolin-Sulfonate, 2,2',6',2"-Terpyridin-Sulfonate oder Porphin-Sulfonate. Weiterhin ist die Verwendung mehrzähniger, insbesondere die Verwendung zwei-, drei- oder vierzähniger, carboxylierter, stickstoffhaltiger Komplexbildner im erfindungsgemäßen Verfahren besonders bevorzugt, insbesondere die Verwendung von 2,2'-Bipyridin-Carboxylaten, 1,10-Phenanthrolin-Carboxylaten, 2,2'-Bichinolin-Carboxylaten, 2,2',6',2"-Terpyridin-Carboxylaten und Porphin-Carboxylaten.

Unter Einwirkung dieser Komplexbildner bilden sich vermutlich über das freie Elektronenpaar der Stickstoffatome koordinative Bindungen mit dem Rhodium-Zentralatom des Rhodium-Katalysators aus, wobei vermutlich ein Teil des an das zentrale Rhodiumatom ies Rhodium-Katalysators gebundenen Kohlenmonoxids unter den Bedingungen der Komplexbildung durch diese Liganden reversibel verdrängt wird. Die Anwesenheit von Sulfonat- oder Carboxylatgruppen in den erfindungsgemäß verwendeten, stickstoffhaltigen Komplexbildnern ist kritisch für die Durchführbarkeit des erfindungsgenäßen Verfahrens. Die Komplexbildner können eine oder mehrere Carboxylat- und/oder Sulfonat-Gruppen pro Molekül enthalten, wobei die Anzahl der Carboxylat- und/oder Sulfonat-Gruppen im Molekül selbstverständlich auch von der Molekülgröße des Komplexbildners und dessen Reaktivität gegenüber Sulfonierungsreagenzien abhängig ist. Beispielsweise werden in der Regel monosulfonierte Pyridine und 2,2'-Bipyridine als Komplexbildner verwendet, wohingegen die sulfonierten Porphine beispielsweise bis zu vier Sulfonat-Gruppen im Molekül enthalten können. Da Carboxylat-Gruppen tragende stickstoffhaltige Komplexbildner vorteilhaft durch die Oxidation der entsprechenden Alkyl-, vorzugsweise Methyl-substituierten Komplexbildner hergestellt werden können, die nach konventionellen chemischen Verfahren leicht zugänglich sind, können die carboxylierten Komplexbildner je nach Molekülgröße 1 bis 6, vorzugsweise 1 bis 4 und besonders bevorzugt 1 bis 3 Carboxylgruppen tragen. Die Anzahl der Carboxyl- und/oder Sulfonsäure-Gruppen im Komplexbildner-Molekül beeinflußt die Wasserlöslichkeit dieser Komplexbildner. Selbstverständlich können im erfindungsgemäßen Verfahren auch stickstoffhaltige Komplexbildner eingesetzt werden, die sowohl Carboxylgruppen als auch Sulfonsäuregruppen als Substituenten enthalten oder Gemische sulfonierter und carboxylgruppenhaltiger Komplexbildner verwendet werden. Die Sulfonat- und Carboxylat-Gruppen liegen in den erfindungsgemäß angewandten Komplexbildnern vorzugsweise in Salzform vor, insbesondere in Form von wasserlöslichen Salzen, besonders bevorzugt in Form ihrer Onium-, Alkalimetall- und/oder Erdalkalimetallsalze. Die Art des verwendeten Oniumsalzes ist im allgemeinen nicht kritisch. So können z.B. Ammonium-, Phosphonium- oder Arsoniumsalze der betreffenden Carbon- oder Sulfonsäuren eingesetzt werden. Zur Vermeidung von Mißverständnissen sei an dieser Stelle ausdrücklich darauf hingewiesen, daß die erfindungsgemäß verwendbaren stickstoffhaltigen Komplexbildner-Sulfonate oder -Carboxylate die Sulfonat- oder Carboxylatgruppen als Substituenten tragen und nicht etwa salzartig mit irgendwelchen Sulfonatoder Carboxylatanionen verbunden sind.

Die genannten Komplexbildner können zusätzlich mit unter den Reaktionsbedingungen inerten Substituenten wie Halogenatomen, insbesondere Fluor-, Chlor- oder Bromatomen, Alkylgruppen, insbesondere C₁- bis C₄-Alkylgruppen, Arylgruppen, insbesondere C₆- bis C₁₀-Arylgruppen, C₇- bis C₁₂-Aralkylgruppen, der Nitrogruppe, der Hydroxygruppe, der Cyanogruppe, Alkoxygruppen, insbesondere C₁- bis C₄-Alkoxygruppen sowie C₁- bis C₁₀-Alkylsulfonatgruppen substituiert sein.

Eine Substitution mit Alkyl-, Aryl- oder Aralkylgruppen kann insbesondere dann von Vorteil sein, wenn der stickstoffhaltige Komplexbildner reaktionsträge ist und nur unter drastischen Bedingungen sulfoniert werden kann. Da carbocyclische Aryl- und Aralkylgruppen oftmals leichter zu sulfonieren sind als elektronenarme, stickstoffhaltige Heterocyclen, wie Pyridin oder Bipyridin, läßt sich im Falle einer derartigen Substitution des Heterocyclus oder im Falle einer Anellierung aromatischer, nicht-heterocyclischer Ringsysteme an den stickstoffhaltigen Heterocyclus die Sulfonierung unter wesentlich milderen Bedingungen ausführen, ohne daß diese derart substituierten, stickstoffhaltigen Komplexbildner durch eine solche Substitution in ihrer Eigenschaft als Komplexbildner für das erfindungsgemäße Verfahren beeinträchtigt werden. Ähnliches gilt auch für den Fall einer Alkylsubstitution des stickstoffhaltigen Komplexbildners, da die aliphatischen Seitenketten im allgemeinen leicht z.B. mittels Sulfurylchlorid in die entsprechenden Sulfochloride umgewandelt werden können, deren basische Hydrolyse die entsprechenden Sulfonate ergibt.

Die sulfonierten stickstoffhaltigen Komplexbildner können aus den entsprechenden, nicht sulfonierten Stammverbindungen nach an sich herkömmlichen Sulfonierungsverfahren, wie der Umsetzung mit konzentrierter Schwefelsäure oder Oleum, gegebenenfalls in Gegenwart von Katalysatoren, wie Quecksilbersulfat, oder durch die Umsetzung dieser Verbindungen mit Halogensulfonsäuren, vorzugsweise mit Chlorsulfonsäure und anschließender Hydrolyse der gebildeten Sulfonsäurehalogenide zu den Sulfonsäuresalzen, erzeugt werden. Alkansulfonat - substituierte Komplexbildner, wie das Pyridin-4-Ethansulfonat, sind z.B. durch die Sulfochlorierung der entsprechenden Stammverbindungen, im zuvor beispielhaft angegebenen Fall, dem 4-Ethylpyridin, mittels Sulfurylchlorid und die anschließende alkalische Hydrolyse des gebildeten Sulfochlorids zugänglich. Diese und andere zur Herstellung der sulfonierten Komplexbildner anwendbare Methoden sind in C. Ferri, Reaktionen der organischen Synthese, S. 165 - 172 und S. 484 - 485, Thieme, Stuttgart 1978 beschrieben. Beispielsweise ist 2,2'-Bipyridin-5-Sulfonsäure durch die Sulfonierung von 2,2'-Bipyridin nach dem Verfahren von Herrmann et al., Chem.Ber. 123, 1953 (1990) erhältlich. 1,10-Phenanthrolin kann auf die analoge Weise sulfoniert werden. Weitere mit Sulfonsäuregruppen substituierte Bipyridine können nach Campa et al., An. Quim., Ser. C 84, 128 (1988) hergestellt werden. Die Terpyridine, die z.B. nach den von Kröhnke in Synthesis 1 (1976) angegebenen Methoden hergestellt werden können, lassen sich ebenfalls nach den von Ferri angegebenen Verfahren in die betreffenden Sulfonate überführen.

Bei der Sulfonierung der stickstoffhaltigen Komplexbildner nach den zuvor angegebenen Methoden werden in der Regel Isomerengemische der sulfonierten Komplexbildner gebildet, die die Sulfonatgruppen an den verschiedenen möglichen Positionen des stickstoffhaltigen Komplexbildner-Ringsystems und/oder an den an das Komplexbildner-Ringsystem gebundenen Substituenten, insbesondere an den aromatischen Substituenten, wie den Phenyl- oder Naphthyl-Substituenten, tragen. Außerdem kommt es, insbesondere bei den komplexeren Komplexbildner-Ringsystemen sowie bei den mit aromatischen Substituenten substituierten Komplexbildnern, häufig zur Mehrfachsulfonierung dieser Komplexbildner. Die so nach den verschiedenen Sulfonierungsmethoden erhaltenen Komplexbildnersulfonat-Gemische, bestehend aus isomeren, einfach sulfonierten Komplexbildnern und mehrfach sulfonierten Komplexbildnern, können selbstverständlich nach den Verfahren des Standes der Technik, wie Kristallisation oder Ionenaustauschchromatographie, in die einzelnen sulfonierten Komponenten zerlegt und die so erhaltenen Einzelkomponenten als Komplexbildner im erfindungsgemäßen Verfahren eingesetzt werden. Da aber der Ort der Sulfonierung als auch der Sulfonierungsgrad der sulfonierten Komplexbildner für das Ergebnis des erfindungsgemäßen Verfahrens im allgemeinen nicht kritisch sind und es für den Erfolg des erfindungsgemäßen Verfahrens im allgemeinen nur darauf ankommt, daß die stickstoffhaltigen Komplexbildner sulfoniert sind, werden im erfindungsgemäßen Verfahren vorzugsweise die nach den verschiedenen Sulfonierungsmethoden erhaltenen Gemische der Komplexbildnersulfonate, vorteilhaft nach deren Überführung in ihre wasserlöslichen Salze, verwendet, wodurch sich die Auftrennung in die sulfonierten Einzelkomponenten des Komplexbildnersulfonatgemisches erübrigt. Aus diesem Grunde wird für den Zweck dieser Anmeldung in der Regel auch nicht von den individuellen Sulfonaten der verschiedenen Komplexbildner gesprochen, sondern stattdessen die betreffenden Komplexbildner gruppenweise bezeichnet, z.B. als Chinolin-Sulfonate, 2,2'-Bichinolin-Sulfonate, 1,10-Phenanthrolin-Sulfonate, Terpyridin-Sulfonate usw. Nur in Ausnahmefällen, wenn bei bestimmten Sulfonierungsverfahren zur Sulfonierung spezieller Komplexbildner der betreffende sulfonierte Komplexbildner unter den üblicherweise angewandten Reaktionsbedingungen praktisch ausschließlich entsteht, wird in dieser Anmeldung namentlich vom individuellen, sulfonierten Komplexbildner gesprochen.

Zur Verdeutlichung der Vielzahl der erfindungsgemäß anwendbaren Komplexbildner werden im folgenden beispielhaft eine Reihe von stickstoffhaltigen Komplexbildnern genannt, die sich nach Sulfonierung, entsprechend den zuvor angegebenen Methoden und Überführung in ihre Salze, im erfindungsgemäßen Verfahren einsetzen lassen: Pyridin, die Picoline, andere Alkylpyridine, Chinolin, 5,6-Benzochinolin, 2,2'-Bipyridin, 2,2'-Bichinolin der Formel I 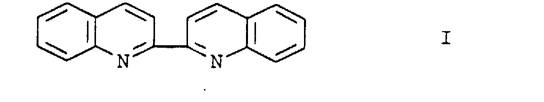 5,6,5',6'-Dibenzo-2,2'-bichinolin der Formel II 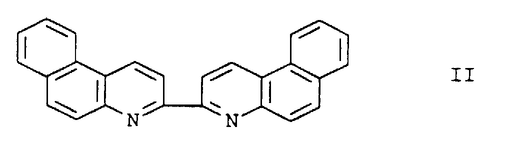 1,10-Phenanthrolin, 2,9-Dimethylphenanthrolin, 4,7-Diphenyl-1,10-phenanthrolin ("Bathophenanthrolin") der Formel III 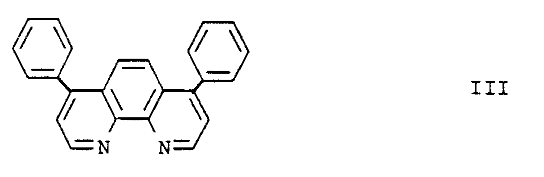 2,9-Dimethyl-4,7-diphenyl-1,10-phenanthrolin ("Bathocuproin") der Formel IV 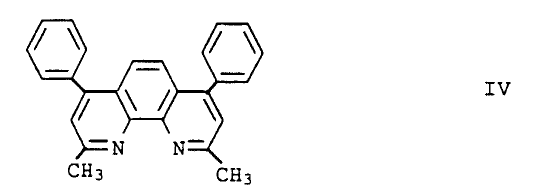 4,5-Diazafluoren V 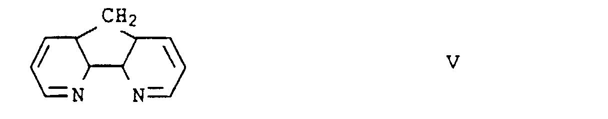 Dipyrido[3,2-a: 2',3'-c]phenazin der Formel VI (Verbindungen V und VI sind erhältlich nach Aust. J. Chem. 23, 1023 (1970)) 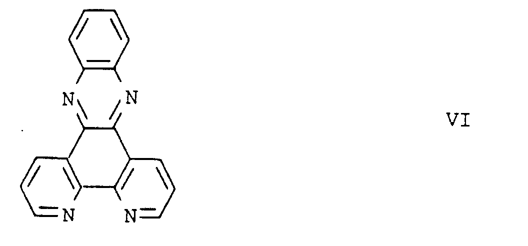 2,2',6',2"-Terpyridin der Formel VII 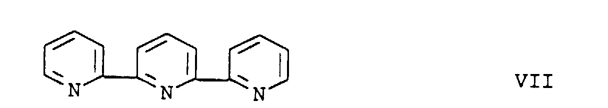 4'-Phenyl-2,2',6',2"-Terpyridin der Formel VIII 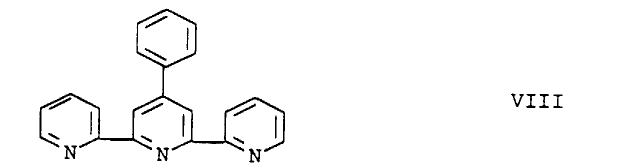 4-Methyl-(4'-phenyl)-(4"-methyl)-2,2',6',2"-terpyridin der Formel IX 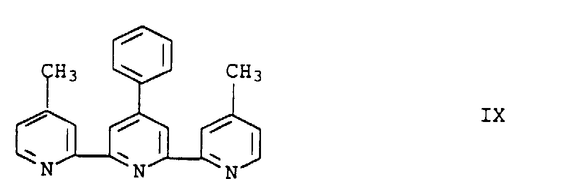 sowie die Porphine.

Als besonders gut geeignet für das erfindungsgemäße Verfahren haben sich die sulfonierten stickstoffhaltigen Komplexbildner erwiesen, deren Eigenschaften als Komplexbildner auf das in den jeweiligen Komplexbildnern enthaltene Strukturelement des 2,2'-Bipyridin-, 1,10-Phenanthrolin- und 2,2',6',2"-Terpyridin-Ringsystems zurückgeführt werden kann, wie es beispielsweise bei den oben genannten Verbindungen der Formeln I bis IX der Fall ist.

Die Carboxylgruppen-tragenden Komplexbildner können aus den 1-bis 6-fach, vorzugsweise 1- bis 4-fach und besonders bevorzugt 1-bis 3-fach Methyl-substituierten Verbindungen I bis IX durch Oxidation der Methylgruppen, beispielsweise mittels Kaliumpermanganat, hergestellt werden. Die mit Methylgruppen substituierten oder benzanellierten Verbindungen I bis IX können beispielsweise nach den Verfahren, wie sie von Kröhnke et al., Synthesis 1 (1976); Sasse et al., J. Chem. Soc. 616 (1956); Sasse et al., J. Heterocycl. Chem. 8, 483 (1971); Bos et al., Synth. Commun. 9, 497 (1979) oder Elliot et al., J. Am. Chem. Soc. 104, 7519 (1982) beschrieben werden, erhalten werden. Die Oxidation der Methylgruppen zu Carboxylgruppen kann z.B. mit Kaliumpermanganat nach den allgemein anwendbaren Methoden wie sie von Anderson et al. (J. Chem. Soc. Dalton Trans. 2247 (1985)) oder Hanabusa et al. (Makromol. Chem. 190, 1 (1989)) beschrieben werden, durchgeführt werden. Von den Carboxylgruppen-tragenden, stickstoffhaltigen Komplexbildnern ist die Verwendung von 2,2'-Bipyridin-4,4'-dicarbonsäure und der 2,2'-Bipyridin-5,5'-dicarbonsäure bzw. von deren Salzen bevorzugt.

Das erfindungsgemäße Verfahren eignet sich besonders gut zur Hydroformylierung von Olefinen mit mehr als 3, vorzugsweise mehr als 7 Kohlenstoffatomen, insbesondere zur Hydroformylierung von C₇- bis C₂₀-Olefinen, die geradkettig oder verzweigt sein können und die α-olefinische und/oder interne Doppelbindungen enthalten können, z.B. Octen-1, Dodecen-1, Trimer- und Tetramerpropylen, oder Dimer-, Trimer- und Tetramerbutylen. Ebenso können ungesättigte Oligomere anderer Olefine hydroformyliert werden. Desgleichen Cooligomere verschiedener Olefine. Die aus diesen Olefinen gebildeten Aldehyde dienen z.B. als Vorstufen für die Herstellung von Weichmacheralkoholen und Tensiden, die daraus auf an sich herkömmliche Weise durch Hydrierung erzeugt werden können. Die zur Hydroformylierung eingesetzten Olefine können z.B. durch die säurekatalysierte Wasserabspaltung aus den entsprechenden Fettalkoholen oder nach einer Vielzahl anderer technischer Verfahren, wie sie beispielsweise in Weissermel, Arpe: Industrielle Organische Chemie, S. 67-86, Verlag Chemie, Weinheim, 1978, dargestellt sind, erhalten werden. Werden im erfindungsgemäßen Verfahren α-Olefine eingesetzt, so können diese wahlweise durch direkten Eintrag in die Hydroformylierungsstufe zu den entsprechenden n-Aldehyden hydroformyliert oder durch deren Eintrag in die Vorcarbonylierungsstufe, nach ihrer Isomerisierung zu internen Olefinen zu Isoaldehyden hydroformyliert werden, auf deren Anwendung bereits hingewiesen wurde.

Besonders gut geeignet ist das erfindungsgemäße Verfahren auch zur Hydroformylierung von polymeren Olefinen, beispielsweise niedermolekularem Polyisobuten, niedermolekularem Polybutadien oder niedermolekularem 1,3-Butadien-Isobuten- oder Buten-Copolymeren. Unter "niedermolekulare Polymere" werden insbesondere Polymere mit Molgewichten von 500 bis 5000 Dalton verstanden. Es können aber auch höhermolekulare, ungesättigte Polymere hydroformyliert werden. Einzige Voraussetzung hierfür ist, daß diese im Hydroformylierungsmedium löslich sind. Die Hydroformylierungsprodukte dieser polymeren Olefine, insbesondere die des niedermolekularen Polyisobutens können beispielsweise nach dem Verfahren der EP-A 244 616 durch reduktive Aminierung in die entsprechenden Amine umgewandelt werden, die als Kraftstoffadditiv Verwendung finden. Niedermolekulares Polyisobuten ist beispielsweise - nach dem Verfahren von EP-A 145 235 erhältlich, niedermolekulare Isobuten-1,3-Butadien-Copolymere können z.B. nach dem Verfahren der deutschen Patentanmeldung P 4306384.5 gewonnen werden.

Das erfindungsgemäße Verfahren eignet sich praktisch zur Herstellung aller Aldehyde, die auf dem Wege der Hydroformylierung von Olefinen erhältlich sind. Insbesondere sei darauf hingewiesen, daß beispielsweise auch substituierte Olefine, die im allgemeinen 1 bis 2, vorzugsweise einen Substituenten, tragen können, nach dem erfindungsgemäßen Verfahren hydroformyliert werden können. Beispielsweise können nach dem erfindungsgemäßen Verfahren ungesättigte, aliphatische Carbonsäureester, Acetale, Alkohole, Ether, Aldehyde, Ketone und Amine und Amide hydroformyliert werden. Als derartige substituierte Ausgangsolefine sind z.B. Methacrylsäureester, Dicyclopentadien, Vinyl- und Allylether, insbesondere entsprechend substituierte Derivate ungesättigter Fettsäuren von Interesse, beispielsweise die Ester der Öl-, Linol-, Linolen-, Ricinol- oder Erucasäure. Die aus diesen olefinischen Rohstoffen durch Hydroformylierung erhältlichen Aldehyde sind ebenfalls Ausgangsmaterialien zur Herstellung biologisch leicht abbaubarer, waschaktiver Substanzen.

Eine weitere Ausgestaltung der vorliegenden Erfindung betrifft Verfahren zur Herstellung verzweigter Carbonsäuren, Alkohole oder Amine aus α-Olefinen, wobei die α-Olefine nach dem erfindungsgemäßen Verfahren in der Vorcarbonylierungsstufe zu internen Olefinen isomerisiert, anschließend zu Isoaldehyden hydroformyliert und die so erhaltenen Isoaldehyde auf an sich herkömmliche Weise zu verzweigten Carbonsäuren oxidiert, zu verzweigten Alkoholen reduziert oder verzweigten Aminen reduktiv aminiert werden.

Die Oxidation der erfindungsgemäß aus α-Olefinen erhaltenen Isoaldehyde oder Isoaldehyd/n-Aldehyd-Gemische kann auf an sich herkömmliche Weise erfolgen, beispielsweise durch die Oxidation der Aldehyde mit Luftsauerstoff oder Sauerstoff gemäß den Verfahren, wie sie z.B. in Ullmann's Encyclopedia of Industrial Chemistry, 5. Aufl., Vol. A5, S. 239, VCH Verlagsgesellschaft, Weinheim, 1986 dargestellt sind.

Die katalytische Hydrierung der nach dem erfindungsgemäßen Verfahren aus α-Olefinen erhältlichen Isoaldehyde oder Isoaldehyd/ n-Aldehydgemische zu verzweigten Alkoholen kann auf an sich bekannte Weise, beispielsweise nach den Verfahren von Ullmann's Encyclopedia of Industrial Chemistry, 5. Aufl., Vol. A1, S. 279, VCH Verlagsgesellschaft, Weinheim, 1985 oder G.H. Ludwig, Hydrocarbon Processing, März 1993, S. 67, bewerkstelligt werden.

Die reduktive Aminierung der nach dem erfindungsgemäßen Verfahren aus α-Olefinen erhältlichen Isoaldehyde oder Isoaldehyd/n-Aldehydgemische kann auf an sich bekannte Weise, beispielsweise nach den Verfahren von Ullmann's Encyclopedia of Industrial Chemistry, 5. Aufl., Vol. A2, S. 1, VCH Verlagsgesellschaft, Weinheim, 1985 erfolgen. Als Ausgangsmaterial zur Herstellung von Aminen können sowohl Ammoniak, primäre C1- bis C20-Amine oder sekundäre C2- bis C20-Amine eingesetzt werden.

### Beispiele

In den folgenden Beispielen steht das Wort "Bipyridin" für das Natriumsalz der 2,2'-Bipyridin-5-sulfonsäure.

### Beispiel 1

### Vorcarbonylierung in Gegenwart von rohem Trimerpropylen-Oxo-Produkt

50 g einer 2,8 gew.-%igen wäßrigen Bipyridinlösung, welche durch Extraktion des rohen Austrags aus der Hydroformylierung von Trimerpropylen mit ca. 100 Gew.-ppm Rhodium angereichert war, wurde zusammen mit 50 ml rohem, Rhodium-freien Trimerpropylen-Oxo-Produkt in einem Druckreaktor bei 130°C und einem Druck von 280 bar CO/H₂ (Molverhältnis 1:1) 1h lang kräftig gerührt. Nach dem Abkühlen wurde der Austrag aus dieser Carbonylierung entspannt, und das erhaltene zweiphasige Gemisch wurde auf seinen Rhodiumgehalt analysiert. Die organische Phase enthielt 55 Gew.- ppm und die wäßrige Phase 6 Gew.-ppm Rhodium.

### Beispiel 2

### Vorcarbonylierung in Gegenwart von Aldehyd

24 ml einer 2,8 gew.-%igen, wäßrigen Bipyridinlösung, welche nach Extraktion von rohem Trimerpropylen-Oxoaustrag mit ca. 100 Gew.- ppm Rhodium angereichert war, wurde zusammen mit 30 ml iso-Decanal in einem Druckreaktor bei 130°C und einem Druck von 280 bar CO/H₂ (Molverhältnis 1:1) 1 h lang kräftig gerührt. Nach dem Abkühlen auf Raumtemperatur wurde der Austrag der Vorcarbonylierung entspannt und das erhaltene zweiphasige Gemisch auf seinen Rhodiumgehalt analysiert. Die organische Phase enthielt 70, die wäßrige Phase 10 Gew.-ppm Rhodium.

### Beispiel 3

### Vorcarbonylierung in Gegenwart von Alkohol

24 ml einer 2,8 gew.-%igen, wäßrigen Bipyridinlösung, welche nach Extraktion von rohem Trimerpropylen-Oxoaustrag mit ca. 100 Gew.- ppm Rhodium angereichert war, wurde zusammen mit 24 g iso-Decanol in einem Druckreaktor bei 130°C und einem Druck von 280 bar CO/H₂ (Molverhältnis 1:1) 1 h lang kräftig gerührt. Nach Abkühlen auf Raumtemperatur und Entspannung wurde das erhaltene zweiphasige Vorcarbonylierungsgemisch auf seinen Rhodiumgehalt untersucht. Die organische Phase enthielt 50, die wäßrige Phase 38 Gew.-ppm Rhodium.

### Beispiel 4

### Vorcarbonylierung mit Kohlenmonoxid in Gegenwart von Olefin

50 g der in den vorausgegangenen Beispielen verwendeten 2,8 gew.-%igen Bipyridinlösung, wurden zusammen mit 100 g Trimerpropylen in einem Druckreaktor bei 130°C und 280 bar CO-Druck 1 h lang kräftig gerührt. Nach Abkühlung auf Raumtemperatur und Entspannung enthielt die organische Phase des Vorcarbonylierungsaustrags 60 und die wäßrige Phase 19 Gew.-ppm Rhodium.

### Beispiel 5

### Vorcarbonylierung und anschließende Hydroformylierung

60 g Trimerpropylen und 30 ml der in den vorausgegangenen Beispielen verwendeten Bipyridinlösung wurden in einem Autoklaven bei 130°C und einem Druck von 280 bar CO/H₂ (Molverhältnis 1:1) 1 h lang kräftig gerührt. Die organische Phase des Vorcarbonylierungsaustrags enthielt 36, die wäßrige 80 Gew.-ppm Rhodium. 19 g der organischen Phase wurden mit weiteren 81 g Trimerpropylen versetzt und im Autoklaven bei 130°C und einem Druck von 280 bar CO/H₂ (Molverhältnis 1:1) 3 h läng hydroformyliert. Es wurden 112,5 g Produkt mit einem Aldehydgehalt von 76 Gew.-% und einem Alkoholgehalt von 3,9 Gew.-% erhalten. 18 % des Trimerpropylens waren nicht umgesetzt.

### Beispiel 6

### Vorcarbonylierung/Isomerisierung und Hydroformylierung eines α-Olefins

68 ml Dodecen-1 und 25 ml der in den vorausgegangenen Beispielen verwendeten Bipyridinlösung, wurden in einem Autoklaven bei 130°C und einem Druck von 280 bar CO 1 h lang kräftig gerührt. Die organische Phase des Vorcarbonylierungsaustrags enthielt 29, die wäßrige Phase 16 Gew.-ppm Rhodium. 32,7 g der organischen Phase wurden in einem Autoklaven bei 90°C und bei 280 bar CO/H₂ (Molverhältnis 1:1) 3 h lang hydroformyliert. Das ausgetragene Hydroformylierungsprodukt enthielt nach gaschromatographischer Analyse neben 1,2 Gew.-% nicht umgesetzten Dodecenen (Isomerengemisch) 98,2 Gew.-% Tridecanale. Der Anteil an n-Tridecanal betrug lediglich 16 Gew.-%. Der Anteil an Methyldodecanal betrug 24,9 Gew.-% und weitere 57,4 Gew.-% bestanden aus einem Gemisch verschiedener, interner Tridecanale.

### Beispiel 7

### Extraktion eines Hydroformylierungsaustrags bei 85°C unter Atmosphärendruck

90 g Oxoaustrag aus der Hydroformylierung von Trimerpropylen mit einem Rhodiumgehalt von 74 Gew.-ppm wurden bei 85°C unter einer Stickstoffatmosphäre bei Atmosphärendruck mit 42 g einer gemäß den Beispielen 1 und 2 an Rhodium abgereicherten wäßrigen Bipyridinlösung versetzt (Rhodiumgehalt: 9,2 Gew.-ppm) und 30 min lang gerührt. Nach Phasentrennung enthielt die organische Phase noch 17, die wäßrige Phase 83 Gew.-ppm Rhodium.

### Beispiel 8

### Mehrstufige Extraktion eines Hydroformylierungsaustrags bei 120°C unter erhöhtem Druck

100 g Oxoaustrag aus der Hydroformylierung von Trimerpropylen mit einem Rhodiumgehalt von 67 Gew.-ppm wurden bei 120°C unter einer Stickstoffatmosphäre bei einem Druck von 3 bar mit 50 g einer gemäß den Beispielen 1 und 2 erhaltenen, an Rhodium abgereicherten Bipyridinlösung (Rhodiumgehalt: 7,4 Gew.-ppm) zweimal unter Rühren 30 min extrahiert. Nach Entspannung und Phasentrennung enthielt die organische Phase weniger als 0,5 Gew.-ppm Rhodium. Der Rhodiumgehalt der wäßrigen Bipyrdinlösung betrug nach der ersten Extraktion 127, nach der zweiten Extraktion 21 Gew.-ppm.

### Beispiel 9

### Mehrstufige Extraktion eines Hydroformylierungsaustrags unter einer Wasserstoffatmosphäre

Analog Beispiel 7 wurden 112 g Oxoaustrag aus der Hydroformylierung von Trimerpropylen mit einem Rhodiumgehalt von 55 Gew.- ppm bei 120°C unter einer Wasserstoffatmosphäre bei 120°C und einem Druck von 4,2 bar mit einer 2,8 gew.-%igen wäßrigen Bipyridinlösung (Rhodiumgehalt: 9,2 Gew.-ppm) zweimal unter Rühren (30 Min) extrahiert. Die organische Phase enthielt nach dieser Prozedur 1,1 Gew.-ppm Rhodium, die wäßrige Phase aus der ersten Extraktion 140 und die zweite wäßrige Phase aus der zweiten Extraktion 16 Gew.-ppm Rhodium.

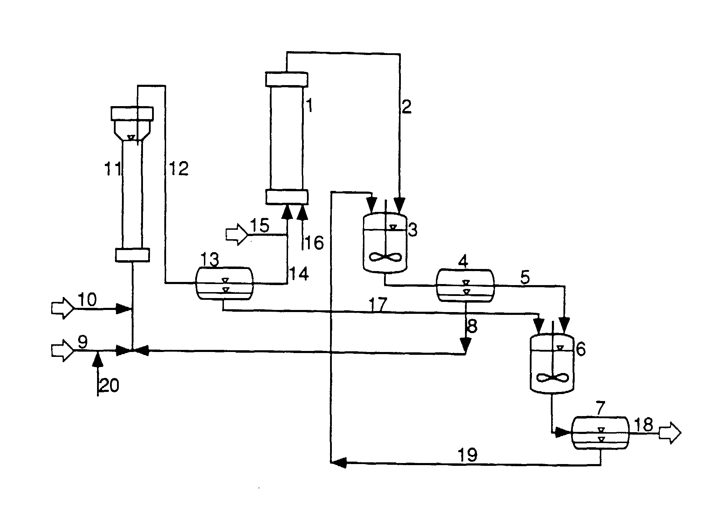

## Patentansprüche

1. Verfahren zur Herstellung von Aldehyden durch die Hydroformylierung von Olefinen mit mehr als 3 Kohlenstoffatomen, umfassend die Stufe der Hydroformylierung mittels eines homogen im Reaktionsmedium gelösten Rhodium-Katalysators, die Abtrennung des Rhodium-Katalysators vom Austrag der Hydroformylierungsreaktion und die Rückführung des vom Hydroformylierungsaustrag abgetrennten Rhodiums in die Hydroformylierungsstufe, dadurch gekennzeichnet, daß man den Rhodium-Katalysator aus dem Hydroformylierungsaustrag mittels einer wäßrigen Lösung eines stickstoffhaltigen Komplexbildners aus der Gruppe der sulfonierten Pyridine, der sulfonierten Chinoline, der gegebenenfalls substituierten, sulfonierten oder sulfonierte Substituenten tragenden 2,2'-Bipyridine, 1,10-Phenanthroline, 2,2'-Bichinoline, 2,2',6',2"-Terpyridine und Porphine, und/oder aus der Gruppe der carboxylierten Pyridine, der carboxylierten Chinoline, der gegebenenfalls substituierten, carboxylierten oder carboxylierte Substituenten tragenden 2,2'-Bipyridine, 1,10-Phenanthroline, 2,2'-Bichinoline, 2,2',6',2''-Terpyridine und Porphine, in die wäßrige Phase extrahiert und aus dem extrahierten Hydroformylierungsaustrag den Alkohol und/oder Aldehyd isoliert, den wäßrigen rhodiumhaltigen Extrakt einer Vorcarbonylierungsstufe zuführt und ihn in der Vorcarbonylierungsstufe in Gegenwart einer im wesentlichen wasserunlöslichen, organischen Flüssigkeit und in Gegenwart von Kohlenmonoxid, Synthesegas oder eines Kohlenmonoxid-haltigen Gasgemischs bei einem Druck von 50 bis 1000 bar und einer Temperatur von 50 bis 180°C einer Vorcarbonylierung unterzieht, den Austrag aus der Vorcarbonylierungsstufe in eine den Hauptteil des Rhodiums enthaltende organische Phase und in eine den Komplexbildner enthaltende wäßrige Phase trennt und die organische Phase zur Hydroformylierung des Olefins bei 50 bis 1000 bar und einer Temperatur von 50 bis 180°C der Hydroformylierungsstufe zuführt.

2. Verfahren nach Anspruch 1, dadurch gekennzeichnet, daß man die Extraktion des Rhodium-Katalysators aus dem Hydroformylierungsaustrag bei Temperaturen von 80 bis 140°C und bei einem Druck von 1 bis 20 bar durchführt.

3. Verfahren nach den Ansprüchen 1 und 2, dadurch gekennzeichnet, daß man die Extraktion des Rhodium-Katalysators einoder mehrstufig durchführt und die wäßrige Lösung des Komplexbildners bei der Extraktion bezüglich des Hydroformylierungsaustrags im Gegenstrom führt.

4. Verfahren nach den Ansprüchen 1 bis 3, dadurch gekennzeichnet, daß man die nach Abtrennung der den Hauptteil des Rhodiums enthaltenden organischen Phase nach der Vorcarbonylierungsstufe erhaltene, das Komplexbildner enthaltende wäßrige Phase zur Extraktion des Rhodium-Katalysators aus dem Hydroformylierungsaustrag verwendet.

5. Verfahren nach den Ansprüchen 1 bis 4, dadurch gekennzeichnet, daß das Verfahren in einer Anlage aus einer Vorcarbonylierungseinheit, einer Hydroformylierungseinheit und einer ein- oder mehrstufig arbeitenden Extraktionseinheit kontinuierlich durchgeführt wird.

6. Verfahren nach den Ansprüchen 1 bis 5, dadurch gekennzeichnet, daß man als Olefine unsubstituierte oder mit 1 oder 2 unter den Reaktionsbedingungen inerten Substituenten substituierte, lineare oder verzweigte α-Olefine oder interne Olefine oder Gemische dieser Olefine in An- oder Abwesenheit eines Lösungsmittels einsetzt.

7. Verfahren nach den Ansprüchen 1 bis 6, dadurch gekennzeichnet, daß man Propen- oder Butenoligomere oder -Cooligomere hydroformyliert.

8. Verfahren nach den Ansprüchen 1 bis 6, dadurch gekennzeichnet, daß man niedermolekulare Polyisobutene, Polybutadiene oder Isobuten-1,3-Butadien-Copolymere hydroformyliert.

9. Verfahren nach den Ansprüchen 1 bis 6, dadurch gekennzeichnet, daß man ungesättigte Fettsäurederivate hydroformyliert.

10. Verfahren nach den Ansprüchen 1 bis 9, dadurch gekennzeichnet, daß man
- den Austrag aus der Hydroformylierungsstufe in einer Extraktionsstufe A mit der wäßrigen Lösung des stickstoffhaltigen Komplexbildners aus Extraktionsstufe C extrahiert,
- dieses Extraktionsgemisch in eine erste wäßrige und eine erste organische Phase trennt,
- die erste wäßrige Phase der Vorcarbonylierungsstufe und die erste organische Phase der Extraktionsstufe C zuführt,
- in der Vorcarbonylierungsstufe das in der ersten wäßrigen Phase enthaltene Rhodium in Gegenwart einer im wesentlichen wasserunlöslichen, organischen Flüssigkeit mit Kohlenmonoxid, Synthesegas oder einem Kohlenmonoxid-enthaltenden Gasgemisch carbonyliert,
- den Austrag aus der Vorcarbonylierungsstufe in einer Phasenabscheidung B in eine zweite organische und eine zweite wäßrige Phase trennt,
- die zweite organische Phase dem Hydroformylierungsreaktor und die zweite wäßrige Phase der Extraktionsstufe C zuführt,
- in der Hydroformylierungsstufe das Olefin in Gegenwart von Synthesegas hydroformyliert,
- die zweite wäßrige Phase zur Extraktion von restlichem Rhodium-Katalysator aus der ersten organischen Phase in Extraktionsstufe C verwendet,
- das Extraktionsgemisch aus Extraktionsstufe C in eine dritte organische Phase und eine dritte wäßrige Phase trennt,
- aus der dritten organischen Phase den Aldehyd und/oder den Alkohol isoliert,
- und die dritte wäßrige Phase zur Extraktion des Rhodium-Katalysators aus dem Hydroformylierungsaustrag in die Extraktionsstufe A zurückführt.

11. Verfahren nach den Ansprüchen 1 bis 10, dadurch gekennzeichnet, daß man in der Vorcarbonylierungsstufe als im wesentlichen, wasserunlösliche organische Flüssigkeit den Rohaustrag aus der Hydroformylierungsstufe verwendet.

12. Verfahren nach den Ansprüchen 1 bis 10, dadurch gekennzeichnet, daß man in der Vorcarbonylierungsstufe als im wesentlichen wasserunlösliche, organische Flüssigkeit einen Aldehyd oder Alkohol verwendet.

13. Verfahren nach Anspruch 12, dadurch gekennzeichnet, daß man den in der Hydroformylierungsstufe gebildeten Aldehyd oder Alkohol nach dessen Reinigung oder ein gereinigtes Gemisch aus diesem Aldehyd und Alkohol verwendet.

14. Verfahren nach den Ansprüchen 1 bis 10, dadurch gekennzeichnet, daß man in der Vorcarbonylierungsstufe als im wesentlichen wasserunlösliche, organische Flüssigkeit, Gemische hochsiedender Kondensationsprodukte von Aldehyden verwendet.

15. Verfahren nach den Ansprüchen 1 bis 10, dadurch gekennzeichnet, daß man in der Vorcarbonylierungsstufe als im wesentlichen wasserunlösliche, organische Flüssigkeit ein Olefin verwendet.

16. Verfahren nach Anspruch 15, dadurch gekennzeichnet, daß man als Olefin das zu hydroformylierende Olefin verwendet.

17. Verfahren nach den Ansprüchen 15 und 16, dadurch gekennzeichnet, daß man ein internes Olefin verwendet.

18. Verfahren nach den Ansprüchen 15 und 16, dadurch gekennzeichnet, daß man ein α-Olefin verwendet.

19. Verfahren nach den Ansprüchen 15 und 18, dadurch gekennzeichnet, daß man die Vorcarbonylierung bei Temperaturen von 100 bis 180°C und einem Druck von 50 bis 1000 bar durchführt.

20. Verfahren nach den Ansprüchen 15, 18 und 19, dadurch gekennzeichnet, daß man die Vorcarbonylierung in Gegenwart von Kohlenmonoxid durchführt.

21. Verfahren nach den Ansprüchen 15, 18 und 19, dadurch gekennzeichnet, daß man die Vorcarbonylierung in Gegenwart von Synthesegas durchführt.

22. Verfahren nach den Ansprüchen 1 bis 21, dadurch gekennzeichnet, daß man als stickstoffhaltigen Komplexbildner ein wasserlösliches Salz der 2,2'-Bipyridin-5-sulfonsäure verwendet.

23. Verfahren nach den Ansprüchen 1 bis 21, dadurch gekennzeichnet, daß man als stickstoffhaltigen Komplexbildner das wasserlösliche Salze eines sulfonierten 1,10-Phenanthrolins verwendet.

24. Verfahren nach den Ansprüchen 1 bis 21, dadurch gekennzeichnet, daß man als stickstoffhaltigen Komplexbildner ein wasserlösliches Salz der 2,2'-Bipyridin-4,4'-dicarbonsäure und/ oder der 2,2'-Bipyridin-5,5'-dicarbonsäure verwendet.

25. Verfahren zur Herstellung von Carbonsäuren aus einem internen Olefin oder einem α-Olefin, dadurch gekennzeichnet, daß das Olefin gemäß den Ansprüchen 1 bis 24 hydroformyliert und den dabei gebildeten Aldehyd nachfolgend in an sich bekannter Weise zur Carbonsäure oxidiert.

26. Verfahren zur Herstellung von Alkoholen aus einem internen Olefin oder einem α-Olefin, dadurch gekennzeichnet, daß man das Olefin gemäß den Ansprüchen 1 bis 24 hydroformyliert und den dabei gebildeten Aldehyd nachfolgend in an sich bekannter Weise zum Alkohol reduziert oder hydriert.

27. Verfahren zur Herstellung von Aminen aus einem internen Olefin oder einem α-Olefin, dadurch gekennzeichnet, daß man das Olefin gemäß den Ansprüchen 1 bis 24 hydroformyliert und den dabei gebildeten Aldehyd oder Alkohol in Gegenwart eines Aminierungskatalysators und Wasserstoff mit Ammoniak, einem primären oder sekundären Amin auf an sich bekannte Weise aminiert.

## Claims

1. A process for the preparation of aldehydes by the hydroformylation of olefins of more than 3 carbon atoms, comprising hydroformylation by means of a rhodium catalyst homogeneously dissolved in the reaction medium, the separation of the rhodium catalyst from the mixture discharged from the hydroformylation reaction and the recycling of the rhodium separated from the discharged hydroformylation mixture to the hydroformylation stage, wherein the rhodium catalyst is extracted from the discharged hydroformylation mixture by means of an aqueous solution of a nitrogen-containing complexing agent selected from the group consisting of the sulfonated pyridines, the sulfonated quinolines, the unsubstituted or substituted 2,2'-bipyridines, 1,10-phenanthrolines, 2,2'-biquinolines, 2,2',6',2"-terpyridines and porphines which are sulfonated or carry sulfonated substituents, or from the group consisting of the carboxylated pyridines, the carboxylated quinolines, the unsubstituted or substituted 2,2'-bipyridines, 1,10-phenanthrolines, 2,2'-biquinolines, 2,2',6',2"-terpyridines and porphines which are carboxylated or carry carboxylated substituents, into the aqueous phase, and the alcohol or aldehyde are isolated from the extracted discharged hydroformylation mixture, the aqueous rhodium-containing extract is fed to a precarbonylation stage and is subjected to precarbonylation in the precarbonylation stage in the presence of an essentially water-insoluble organic liquid and in the presence of carbon monoxide, synthesis gas or a carbon monoxide-containing gas mixture at from 50 to 1000 bar and from 50 to 180°C, the mixture discharged from the precarbonylation stage is separated into an organic phase containing the main part of the rhodium and into an aqueous phase containing the complexing agent, and the organic phase is fed to the hydroformylation stage for hydroformylation of the olefin at from 50 to 1000 bar and from 50 to 180°C.

2. A process as claimed in claim 1, wherein the extraction of the rhodium catalyst from the discharged hydroformylation mixture is carried out at from 80 to 140°C and from 1 to 20 bar.

3. A process as claimed in claims 1 and 2, wherein the extraction of the rhodium catalyst is carried out in one or more stages, and the aqueous solution of the complexing agent is fed countercurrent to the discharged hydroformylation mixture in the extraction.

4. A process as claimed in any of claims 1 to 3, wherein the aqueous phase containing the complexing agent and obtained after the precarbonylation stage after separating off the organic phase containing the main part of the rhodium is used for extracting the rhodium catalyst from the discharged hydroformylation mixture.

5. A process as claimed in any of claims 1 to 4, wherein the process is carried out continuously in a plant comprising a precarbonylation unit, a hydroformylation unit and a one-stage or multistage extraction unit.

6. A process as claimed in any of claims 1 to 5, wherein the olefins used are straight-chain or branched α-olefins, which are unsubstituted or substituted by 1 or 2 substituents which are inert under the reaction conditions, or internal olefins or mixtures of these olefins, in the presence or absence of a solvent.

7. A process as claimed in any of claims 1 to 6, wherein propene or butene oligomers or cooligomers are hydroformylated.

8. A process as claimed in any of claims 1 to 6, wherein low molecular weight polyisobutenes, polybutadienes or isobutene/1,3-butadiene copolymers are hydroformylated.

9. A process as claimed in any of claims 1 to 6, wherein unsaturated fatty acid derivatives are hydroformylated.

10. A process as claimed in any of claims 1 to 9, wherein
- the mixture discharged from the hydroformylation stage is extracted in an extraction stage A with the aqueous solution of the nitrogen-containing complexing agent from extraction stage C,
- this extraction mixture is separated into a first aqueous and a first organic phase,
- the first aqueous phase is fed to the precarbonylation stage and the first organic phase to the extraction stage C,
- in the precarbonylation stage, the rhodium contained in the first aqueous phase is carbonylated with carbon monoxide, synthesis gas or a carbon monoxide-containing gas mixture in the presence of an essentially water-insoluble, organic liquid,
- the mixture discharged from the precarbonylation stage is separated in a phase separation B into a second organic and a second aqueous phase,
- the second organic phase is fed to the hydroformylation reaction and the second aqueous phase to the extraction stage C,
- in the hydroformylation stage, the olefin is hydroformylated in the presence of synthesis gas,
- the second aqueous phase is used for extracting residual rhodium catalyst from the first organic phase in extraction stage C,
- the extraction mixture from extraction stage C is separated into a third organic phase and a third aqueous phase,
- the aldehyde or the alcohol is isolated from the third organic phase,
- and the third aqueous phase is recycled to the extraction stage A for extracting the rhodium catalyst from the discharged hydroformylation mixture.

11. A process as claimed in any of claims 1 to 10, wherein the crude mixture discharged from the hydroformylation stage is used in the precarbonylation stage as the essentially water-insoluble organic liquid.

12. A process as claimed in any of claims 1 to 10, wherein an aldehyde or alcohol is used in the precarbonylation stage as the essentially water-insoluble organic liquid.

13. A process as claimed in claim 12, wherein the aldehyde or alcohol formed in the hydroformylation stage is purified and then used, or a purified mixture of this aldehyde and alcohol is used.

14. A process as claimed in any of claims 1 to 10, wherein a mixture of high-boiling condensation products of aldehydes is used in the precarbonylation stage as the essentially water-insoluble organic liquid.

15. A process as claimed in any of claims 1 to 10, wherein an olefin is used in the precarbonylation stage as the essentially water-insoluble organic liquid.

16. A process as claimed in claim 15, wherein the olefin used is the olefin to be hydroformylated.

17. A process as claimed in claims 15 and 16, wherein an internal olefin is used.

18. A process as claimed in claims 15 and 16, wherein an α-olefin is used.

19. A process as claimed in claims 15 and 18, wherein the precarbonylation is carried out at from 100 to 180°C and from 50 to 1000 bar.

20. A process as claimed in claims 15, 18 and 19, wherein the precarbonylation is carried out in the presence of carbon monoxide.

21. A process as claimed in claims 15, 18 and 19, wherein the precarbonylation is carried out in the presence of synthesis gas.

22. A process as claimed in any of claims 1 to 21, wherein the nitrogen-containing complexing agent used is a water-soluble salt of 2,2'-bipyridine-5-sulfonic acid.

23. A process as claimed in any of claims 1 to 21, wherein the nitrogen-containing complexing agent used is the water-soluble salt of a sulfonated 1,10-phenanthroline.

24. A process as claimed in any of claims 1 to 21, wherein the nitrogen-containing complexing agent used is a water-soluble salt of 2,2'-bipyridine-4,4'-dicarboxylic acid or of 2,2'-bipyridine-5,5'-dicarboxylic acid.

25. A process for the preparation of a carboxylic acid from an internal olefin or an α-olefin, wherein the olefin is hydroformylated as claimed in any of claims 1 to 24, and the aldehyde formed is subsequently oxidized to the carboxylic acid in a manner known per se.

26. A process for the preparation of an alcohol from an internal olefin or an α-olefin, wherein the olefin is hydroformylated as claimed in any of claims 1 to 24, and the aldehyde formed is subsequently reduced or hydrogenated to the alcohol in a manner known per se.

27. A process for the preparation of an amine from an internal olefin or an α-olefin, wherein the olefin is hydroformylated as claimed in any of claims 1 to 24, and the aldehyde or alcohol formed is aminated with ammonia or a primary or secondary amine in the presence of an amination catalyst and hydrogen in a manner known per se.

## Revendications

1. Procédé de préparation d'aldéhydes par hydroformylation d'oléfines avec plus de 3 atomes de carbone, comprenant l'étape de l'hydroformylation au moyen d'un catalyseur au rhodium mis en solution homogène dans le milieu réactionnel, la séparation du catalyseur au rhodium de l'effluent de la réaction d'hydroformylation et le réacheminement du rhodium séparé de l'effluent d'hydroformylation dans l'étape d'hydroformylation, caractérisé en ce qu'on extrait le catalyseur au rhodium de l'effluent d'hydroformylation au moyen d'une solution aqueuse d'un agent complexant contenant de l'azote choisi dans le groupe des pyridines sulfonées, des quinolines sulfonées, des 2,2'-bipyridines, des 1,10-phénanthrolines, des 2,2'-biquinolines, des 2,2',6',2"-terpyridines et des porphines éventuellement substituées, sulfonées ou portant des substituants sulfonés, et/ou dans le groupe constitué des pyridines carboxylées, des quinolines carboxylées, des 2,2'-bipyridines, des 1,10-phénanthrolines, des 2,2'-biquinolines, des 2,2',6',2"-terpyridines et des porphines éventuellement substituées, carboxylées ou portant des substituants carboxylés, dans la phase aqueuse et en ce qu'on isole de l'effluent d'hydroformylation extrait, l'alcool et/ou l'aldéhyde, en ce qu'on achemine l'extrait aqueux contenant du rhodium vers une étape de précarbonylation et en ce qu'on lui fait subir une précarbonylation dans une étape de précarbonylation en présence d'un liquide organique essentiellement insoluble dans l'eau et en présence de monoxyde de carbone, de gaz de synthèse ou d'un mélange gazeux contenant du monoxyde de carbone à une pression de 50 à 1.000 bars et à une température de 50 à 180 °C, en ce qu'on sépare l'effluent provenant de l'étape de précarbonylation en une phase organique contenant la majeure partie du rhodium et en une phase aqueuse contenant l'agent complexant et en ce qu'on achemine la phase organique en vue de l'hydroformylation de l'oléfine à 50 à 1.000 bars et à une température de 50 à 180 °C vers l'étape d'hydroformylation.

2. Procédé selon la revendication 1, caractérisé en ce qu'on effectue l'extraction du catalyseur au rhodium de l'effluent d'hydroformylation à des températures de 80 à 140 °C et à une pression de 1 à 20 bars.

3. Procédé selon les revendications 1 et 2, caractérisé en ce qu'on effectue l'extraction du catalyseur au rhodium en une ou en plusieurs étapes et en ce qu'on fait passer la solution aqueuse de l'agent complexant lors de l'extraction en contre-courant par rapport à l'effluent d'hydroformylation.

4. Procédé selon les revendications 1 à 3, caractérisé en ce qu'on utilise la phase aqueuse contenant l'agent complexant obtenu après la séparation de la phase organique contenant la majeure partie du rhodium après l'étape de précarbonylation, pour l'extraction du catalyseur au rhodium de l'effluent d'hydroformylation.

5. Procédé selon les revendications 1 à 4, caractérisé en ce que le procédé est effectué en continu dans une installation constituée d'une unité de précarbonylation, d'une unité d'hydroformylation et d'une unité d'extraction opérée en une ou en plusieurs étapes.

6. Procédé selon les revendications 1 à 5, caractérisé en ce qu'on utilise à titre d'oléfines des α-oléfines ou des oléfines internes, linéaires ou ramifiées, non-substituées ou substituées par un ou deux substituants inertes dans les conditions de réaction, ou des mélanges de ces oléfines en absence ou en présence d'un solvant.

7. Procédé selon les revendications 1 à 6, caractérisé en ce qu'on hydroformyle des oligomères ou des co-oligomères de propène ou de butène.

8. Procédé selon les revendications 1 à 6, caractérisé en ce qu'on hydroformyle des polyisobutènes, des polybutadiènes ou des copolymères d'isobutène-1,3-butadiène inférieurs.

9. Procédé selon les revendications 1 à 6, caractérisé en ce qu'on hydroformyle des dérivés d'acides gras insaturés.

10. Procédé selon les revendications 1 à 9, caractérisé en ce que
- on extrait l'effluent provenant de l'étape d'hydroformylation dans une étape d'extraction A avec une solution aqueuse de l'agent complexant contenant de l'azote provenant de l'étape d'extraction C,
- on sépare ce mélange d'extraction en une première phase aqueuse et en une première phase organique,
- on achemine la première phase aqueuse vers l'étape de précarbonylation et la première phase organique vers l'étape d'extraction C,
- on carbonyle, dans une étape de précarbonylation, le rhodium contenu dans la première phase aqueuse, en présence d'un liquide organique essentiellement insoluble dans l'eau, avec du monoxyde de carbone, du gaz de synthèse ou un mélange gazeux contenant du monoxyde de carbone,
- on sépare l'effluent provenant de l'étape de précarbonylation au cours d'une séparation des phases B en une deuxième phase organique et en une deuxième phase aqueuse,
- on achemine la deuxième phase organique vers le réacteur d'hydroformylation et la deuxième phase aqueuse vers l'étape d'extraction C,
- on hydroformyle, à l'étape d'hydroformylation, l'oléfine en présence de gaz de synthèse,
- on utilise la deuxième phase aqueuse pour l'extraction du catalyseur au rhodium résiduel de la première phase organique à l'étape d'extraction C,
- on sépare le mélange d'extraction provenant de l'étape d'extraction C en une troisième phase organique et en une troisième phase aqueuse,
- on isole l'aldéhyde et/ou l'alcool de la troisième phase organique,
- et on réachemine la troisième phase aqueuse en vue de l'extraction du catalyseur au rhodium de l'effluent d'hydroformylation dans l'étape d'extraction A.

11. Procédé selon les revendications 1 à 10, caractérisé en ce qu'on utilise dans l'étape de précarbonylation à titre de liquide organique essentiellement insoluble dans l'eau, l'effluent brut provenant de l'étape d'hydroformylation.

12. Procédé selon les revendications 1 à 10, caractérisé en ce qu'on utilise dans l'étape de précarbonylation à titre de liquide organique essentiellement insoluble dans l'eau, un aldéhyde ou un alcool.

13. Procédé selon la revendication 12, caractérisé en ce qu'on utilise l'aldéhyde ou l'alcool formé à l'étape d'hydroformylation après sa purification, ou un mélange purifié de cet aldéhyde et de cet alcool.

14. Procédé selon les revendications 1 à 10, caractérisé en ce qu'on utilise dans l'étape de précarbonylation à titre de liquide organique essentiellement insoluble dans l'eau, des mélanges de produits de condensation d'aldéhydes à point d'ébullition élevé.

15. Procédé selon les revendications 1 à 10, caractérisé en ce qu'on utilise dans l'étape de précarbonylation à titre de liquide organique essentiellement insoluble dans l'eau, une oléfine.

16. Procédé selon la revendication 15, caractérisé en ce qu'on utilise à titre d'oléfine, l'oléfine à hydroformyler.

17. Procédé selon les revendications 15 et 16, caractérisé en ce qu'on utilise une oléfine interne.

18. Procédé selon les revendications 15 et 16, caractérisé en ce qu'on utilise une α-oléfine.

19. Procédé selon les revendications 15 et 18, caractérisé en ce qu'on effectue la précarbonylation à des températures de 100 à 180°C et à une pression de 50 à 1.000 bars.

20. Procédé selon les revendications 15, 18 et 19, caractérisé en ce qu'on effectue la précarbonylation en présence de monoxyde de carbone.

21. Procédé selon les revendications 15, 18 et 19, caractérisé en ce qu'on effectue la précarbonylation en présence de gaz de synthèse.

22. Procédé selon les revendications 1 à 21, caractérisé en ce qu'on utilise à titre d'agents complexants contenant de l'azote, un sel soluble dans l'eau de l'acide 2,2'-bipyridine-5-sulfonique.

23. Procédé selon les revendications 1 à 21, caractérisé en ce qu'on utilise à titre d'agents complexants contenant de l'azote, des sels solubles dans l'eau d'une 1,10-phénanthroline sulfonée.

24. Procédé selon les revendications 1 à 21, caractérisé en ce qu'on utilise à titre d'agents complexants contenant de l'azote, un sel soluble dans l'eau de l'acide 2,2'-bipyridine-4,4'-dicarboxylique et/ou de l'acide 2,2'-bipyridine-5,5'-dicarboxylique.

25. Procédé pour la préparation d'acides carboxyliques à partir d'une oléfine interne ou d'une α-oléfine, caractérisé en ce qu'on hydroformyle l'oléfine selon les revendications 1 à 24 et en ce qu'on oxyde ensuite l'aldéhyde résultant d'une manière connue en soi en acide carboxylique.

26. Procédé pour la préparation d'alcools à partir d'une oléfine interne ou d'une α-oléfine, caractérisé en ce qu'on hydroformyle l'oléfine selon les revendications 1 à 24 et en ce qu'on réduit ou hydrogène ensuite l'aldéhyde résultant d'une manière connue en soi en alcool.

27. Procédé pour la préparation d'amines à partir d'une oléfine interne ou d'une α-oléfine, caractérisé en ce qu'on hydroformyle l'oléfine selon les revendications 1 à 24 et en ce qu'on amine ensuite l'aldéhyde ou l'alcool résultant en présence d'un catalyseur d'amination et d'hydrogène avec de l'ammoniac, une amine primaire ou une amine secondaire d'une manière connue en soi.
